# EUROPEAN PATENT APPLICATION

(11) **EP 4 830 676 A1**
(43) Date of publication of application: **28.10.2026**
(21) Application number: 25171598.3
(22) Date of filing: 22.04.2025
(51) Int. Cl.: A61F 7/00, A61F 7/02

(54) **A METHOD FOR INDUCING THERMOGENESIS IN A SUBJECT**

(71) Applicant: The Provost, Fellows, Foundation Scholars, & the Other Members of Board, of the College of the Holy & Undiv. Trinit, Dublin 2 Dublin (IE)
(72) Inventor: The designation of the inventor has not yet been filed
(74) Representative: FRKelly

(57) **Abstract**

The present invention relates to inducing thermogenesis in a subject. The invention finds utility in treating hypothermia, inducing weight loss (including non-therapeutic or cosmetic weight loss), treating obesity, treating cancer, and/or treating a metabolic disorder in a subject.

## Description

### Field of the invention

The present invention relates to inducing thermogenesis in a subject. The invention finds utility in treating hypothermia, inducing weight loss (including non-therapeutic or cosmetic weight loss), treating obesity, treating cancer, and/or treating a metabolic disorder in a subject.

### Background to the invention

The brain and the body are intrinsically connected, and interact bi-directionally to maintain homeostasis. While brain/body interactions are now a central topic in neuroscience, only recently have studies shown that mental states can directly influence bodily function, and that associative learning processes can modify peripheral immune and neuroendocrine responses.

When faced with an environmental thermal challenge, birds and mammals actively maintain their body temperature within a narrow range to preserve critical cellular functions. Many brain regions and neural pathways have been implicated in real time thermoregulation, including the lateral hypothalamus (LHA), the medial preoptic area (MPO), and the xiphoid nucleus. The brain triggers several thermoregulatory behavioral responses that maintain homeostatic body temperature, including motivated goal-directed behavioral responses, such as moving to a warm place, nest building, postural changes; as well as autonomic physiological mechanisms such as shivering, vasoconstriction, and non-shivering thermogenesis. The neutrally-regulated metabolic process of non-shivering thermogenesis contributes to the production of heat and the maintenance of core body temperature, and is largely influenced by altered metabolism in brown adipose tissue (BAT).

The enduring physical change in the brain that accounts for a specific memory can be referred to as an 'engram'. Previous studies have identified memory engrams by combining immediate-early gene (i.e. *c-Fos* or *Arc*) tagging methods with optogenetics and chemogenetics. Engram cells can be operationally defined as neuronal ensembles that are activated by learning, undergo physical changes, and whose reactivation leads to the retrieval of the target memory.

A direct link between memory of an experience and immediate physiological changes in the body for host adaptability has yet to be identified.

It would be useful to regulate the production of heat and to alter homeostatic body temperature without the need to expose a subject to the disadvantageous effects (such as stress) of an environmental thermal (cold) challenge or without the need to induce thermogenesis by non-natural means (such as chemical or adrenergic stimulation).

### Summary of the invention

According to a first aspect of the present invention, there is provided a method for inducing thermogenesis in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature.

According to a second aspect of the present invention, there is provided a method for treating hypothermia in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature and treating the hypothermia.

According to a third aspect of the present invention, there is provided a method for inducing weight loss in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature and inducing weigh loss in the subject.

According to a fourth aspect of the present invention, there is provided a non-therapeutic method for inducing weight loss in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature and inducing weigh loss in the subject.

According to a fifth aspect of the present invention, there is provided a cosmetic method for inducing weight loss in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature and inducing weigh loss in the subject.

According to a sixth aspect of the present invention, there is provided a method for treating obesity in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature and treating the obesity.

According to a seventh aspect of the present invention, there is provided a method for treating cancer in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature and treating the cancer.

Optionally, the cancer is a cancer selected from colorectal, tracheal, bronchial, lung, esophageal, breast, liver, pancreatic, gastrointestinal tract, and endometrial cancer.

According to an eighth aspect of the present invention, there is provided a method for treating a metabolic disorder in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature and treating the metabolic disorder.

Optionally, the metabolic disorder is selected from acid-base imbalance, a metabolic brain disease, a disorder of calcium metabolism, a DNA-repair-deficiency disorder, a glucose metabolism disorder, hyperlactatemia, an iron metabolism disorder, a lipid metabolism disorder, malabsorption syndromes, metabolic syndrome X, inborn error of metabolism, a mitochondrial disease, a phosphorus metabolism disorder, porphyria, a proteostasis deficiency, a metabolic skin disease, wasting syndrome and a water-electrolyte imbalance.

Optionally, the method comprises the steps of:
(a) providing to the subject a first temperature below 21deg°C;
(b) simultaneously providing to the subject a stimulus at the first temperature; and
(c) providing to the subject the stimulus at a second temperature different to the first temperature;
thereby inducing thermogenesis in the subject at the second temperature.

Optionally, the first temperature is below 20deg°C, optionally below 19deg°C, optionally below 18deg°C, optionally below 17deg°C, optionally below 16deg°C, optionally below 15deg°C, optionally below 14deg°C, optionally below 13deg°C, optionally below 12deg°C, optionally below 11deg°C, optionally below 10deg°C, optionally below 9deg°C, optionally below 8deg°C, optionally below 7deg°C, optionally below 6deg°C, optionally below 5deg°C, optionally below 4deg°C, optionally below 3deg°C, optionally below 2deg°C, optionally below 1deg°C.

Preferably, the first temperature is below 5deg°C.

Optionally, the first temperature is 0-20deg°C, optionally 0-19deg°C, optionally 0-18deg°C, optionally 0-17deg°C, optionally 0-16deg°C, optionally 0-15deg°C, optionally 0-14deg°C, optionally 0-13deg°C, optionally 0-12deg°C, optionally 0-11deg°C, optionally 0-10deg°C, optionally 0-9deg°C, optionally 0-8deg°C, optionally 1-7deg°C, optionally 2-6deg°C, optionally 3-5deg°C.

Preferably, the first temperature is 3-5deg°C. Further preferably, the first temperature is 4deg°C.

Optionally, the exposing the subject to a first temperature step comprises decreasing the temperature. Further optionally, the exposing the subject to a first temperature step comprises decreasing the ambient temperature.

Optionally, the exposing the subject to a first temperature step comprises decreasing the temperature at a rate of 2deg°C, optionally 4deg°C, optionally 6deg°C, optionally 8deg°C, optionally 10deg°C, optionally 12deg°C, optionally 14deg°C, optionally 16deg°C, optionally 18deg°C, optionally 20deg°C per hour. Further optionally, the exposing the subject to a first temperature step comprises decreasing the ambient temperature at a rate of 2deg°C, optionally 4deg°C, optionally 6deg°C, optionally 8deg°C, optionally 10deg°C, optionally 12deg°C, optionally 14deg°C, optionally 16deg°C, optionally 18deg°C, optionally 20deg°C per hour.

Optionally, the exposing the subject to a first temperature step occurs until the temperature of the subject decreases. Further optionally, the exposing the subject to a first temperature step occurs until the body temperature of the subject decreases. Still further optionally, the exposing the subject to a first temperature step occurs until the average/mean body temperature of the subject decreases. Still further optionally, the exposing the subject to a first temperature step occurs until the average/mean core body temperature of the subject decreases.

Optionally, the temperature of the subject decreases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C. Further optionally, the body temperature of the subject decreases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C. Still further optionally, the average/mean body temperature of the subject decreases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C. Still further optionally, the average/mean core body temperature of the subject decreases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C.

Optionally, the exposing the subject to a first temperature step occurs for at least 1 hour, optionally at least 2 hours, optionally at least 3 hours, optionally at least 4 hours, optionally at least 5 hours, optionally at least 6 hours, optionally at least 7 hours, optionally at least 8 hours, optionally at least 10 hours, optionally at least 12 hours, optionally at least 14 hours, optionally at least 16 hours, optionally at least 20 hours, optionally at least 24 hours.

Preferably, the exposing the subject to a first temperature step occurs for at least 6 hours. Further preferably, the exposing the subject to a first temperature step occurs for at least 18 hours. Still further preferably, the exposing the subject to a first temperature step occurs for at least 18 hours over a period of 3 days.

Optionally, the stimulus is selected from a visual stimulus, an auditory stimulus, a somatosensory stimulus, an olfactory stimulus, a gustatory stimulus, and combinations thereof.

Optionally, the stimulus is a visual indicia. Further optionally, the stimulus is a visual pattern.

Optionally or additionally, the stimulus is a change in luminous flux of a light source. Further optionally or additionally, the stimulus is an increase in luminous flux of a light source. Alternatively, the stimulus is a decrease in luminous flux of a light source.

Optionally or additionally, the stimulus is a change in a structural environment of the subject. Further optionally or additionally, the stimulus is a change in a layout of the structural environment of the subject.

Optionally or additionally, the stimulus is an odor. Further optionally or additionally, the stimulus is provision of an odor.

Optionally, the odor is an acid. Further optionally, the odor is acetic acid (ethanoic acid). Still further optionally, the odor is an acid solution. Still further optionally, the odor is an aqueous acid solution.

Optionally, the odor is trimethylthiazole (2,4,5-Trimethyl-4,5-dihydro-1,3-thiazole).

Optionally or additionally, the stimulus is contact with an electrical current.

Optionally, the exposing the subject to a first temperature step occurs for the same period as the simultaneously exposing the subject to a stimulus at the first temperature step.

Optionally, the exposing the subject to a first temperature step occurs for a different period as the simultaneously exposing the subject to a stimulus at the first temperature step. Further optionally, the exposing the subject to a first temperature step occurs for a longer period then the simultaneously exposing the subject to a stimulus at the first temperature step.

Optionally, the simultaneously exposing the subject to a stimulus at the first temperature step occurs for at least 1 hour, optionally at least 2 hours, optionally at least 3 hours, optionally at least 4 hours, optionally at least 5 hours, optionally at least 6 hours, optionally at least 7 hours, optionally at least 8 hours, optionally at least 10 hours, optionally at least 12 hours, optionally at least 14 hours, optionally at least 16 hours, optionally at least 20 hours, optionally at least 24 hours.

Optionally, the second temperature is above the first temperature.

Optionally, the second temperature is at least 1deg°C above the first temperature. Further optionally, the second temperature is at least 2deg°C, optionally at least 3deg°C, optionally at least 4deg°C, optionally at least 5deg°C, optionally at least 6deg°C, optionally at least 7deg°C, optionally at least 8deg°C, optionally at least 9deg°C, optionally at least 10deg°C, optionally at least 11deg°C, optionally at least 12deg°C, optionally at least 13deg°C, optionally at least 14deg°C, optionally at least 15deg°C, optionally at least 16deg°C, optionally at least 17deg°C, optionally at least 18deg°C, optionally at least 19deg°C, optionally at least 20deg°C, optionally at least 21deg°C above the first temperature.

Optionally, the second temperature is 1-21deg°C above the first temperature. Further optionally, the second temperature is 2-21deg°C, optionally 3-21deg°C, optionally 4-21deg°C, optionally 5-21deg°C, optionally 6-21deg°C, optionally 7-21deg°C, optionally 8-21deg°C, optionally 9-21deg°C, optionally 10-21deg°C, optionally 11-21deg°C, optionally 12-21deg°C, optionally 13-21deg°C, optionally 14-21deg°C, optionally 15-20deg°C, optionally 16-19deg°C, optionally 17-18deg°C, optionally 18deg°C above the first temperature.

Preferably, the second temperature is 18deg°C above the first temperature.

Optionally, the exposing the subject to the stimulus at a second temperature step occurs for at least 1 hour, optionally at least 2 hours, optionally at least 3 hours, optionally at least 4 hours, optionally at least 5 hours, optionally at least 6 hours, optionally at least 7 hours, optionally at least 8 hours, optionally at least 10 hours, optionally at least 12 hours, optionally at least 14 hours, optionally at least 16 hours, optionally at least 20 hours, optionally at least 24 hours.

Optionally, the thermogenesis occurs until the temperature of the subject increases. Further optionally, the thermogenesis occurs until the body temperature of the subject increases. Still further optionally, the thermogenesis occurs until the average/mean body temperature of the subject increases. Still further optionally, the thermogenesis occurs until the average/mean core body temperature of the subject increases.

Optionally, the temperature of the subject increases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C. Further optionally, the body temperature of the subject increases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C. Still further optionally, the average/mean body temperature of the subject increases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C. Still further optionally, the average/mean core body temperature of the subject increases by at least 0.1deg°C, optionally at least 0.2deg°C, optionally at least 0.3deg°C, optionally at least 0.4deg°C, optionally at least 0.5deg°C, optionally at least 0.6deg°C, optionally at least 0.7deg°C, optionally at least 0.8deg°C, optionally at least 0.9deg°C, optionally at least 1.0deg°C, optionally at least 1.2deg°C, optionally at least 1.3deg°C, optionally at least 1.4deg°C, optionally at least 1.5deg°C, optionally at least 1.6deg°C, optionally at least 1.7deg°C, optionally at least 1.8deg°C, optionally at least 1.9deg°C, optionally at least 2.0deg°C.

Preferably, the method comprises the steps of:
(a) exposing the subject to a first temperature of 4deg°C;
(b) simultaneously exposing the subject to a stimulus at the first temperature of 4deg°C; and
(c) exposing the subject to the stimulus at a second temperature of 21deg°C;
thereby inducing thermogenesis in the subject at the second temperature of 21deg°C.

Optionally, the method comprises the additional step of exposing the subject to a temperature of or above 21deg°C. Further optionally, the method comprises the additional step of exposing the subject to a temperature of or above 21deg°C prior to the step of exposing the subject to a first temperature below 21deg°C.

Optionally, the method comprises the steps of:
(a) exposing the subject to a temperature of or above 21deg°C;
(b) exposing the subject to a first temperature below 21deg°C;
(c) simultaneously exposing the subject to a stimulus at the first temperature; and
(d) exposing the subject to the stimulus at a second temperature different to the first temperature;
thereby inducing thermogenesis in the subject at the second temperature.

Optionally, the step of exposing the subject to a temperature of or above 21deg°C occurs for at least 1 hour, optionally at least 2 hours, optionally at least 3 hours, optionally at least 4 hours, optionally at least 5 hours, optionally at least 6 hours, optionally at least 7 hours, optionally at least 8 hours, optionally at least 10 hours, optionally at least 12 hours, optionally at least 14 hours, optionally at least 16 hours, optionally at least 20 hours, optionally at least 24 hours.

Preferably, the exposing the subject to a temperature of or above 21deg°C step occurs for at least 6 hours. Further preferably, the exposing the subject to a temperature of or above 21deg°C step occurs for at least 18 hours. Still further preferably, the exposing the subject to a temperature of or above 21deg°C step occurs for at least 18 hours over a period of 3 days.

Optionally, the method further comprises the additional step of exposing the subject to a temperature of or above 21deg°C. Further optionally, the method further comprises the additional step of exposing the subject to a temperature of or above 21deg°C prior to the step of exposing the subject to the stimulus at a second temperature different to the first temperature.

Optionally, the method comprises the steps of:
(a) exposing the subject to a first temperature below 21deg°C;
(b) simultaneously exposing the subject to a stimulus at the first temperature;
(c) exposing the subject to a temperature of or above 21deg°C; and
(d) exposing the subject to the stimulus at a second temperature different to the first temperature;
thereby inducing thermogenesis in the subject at the second temperature.

Further optionally, the method comprises the steps of:
(a) exposing the subject to a temperature of or above 21deg°C;
(b) exposing the subject to a first temperature below 21deg°C;
(c) simultaneously exposing the subject to a stimulus at the first temperature;
(d) exposing the subject to a temperature of or above 21deg°C; and
(e) exposing the subject to the stimulus at a second temperature different to the first temperature;
thereby inducing thermogenesis in the subject at the second temperature.

Optionally, the further step of exposing the subject to a temperature of or above 21deg°C occurs for at least 1 hour, optionally at least 2 hours, optionally at least 3 hours, optionally at least 4 hours, optionally at least 5 hours, optionally at least 6 hours, optionally at least 7 hours, optionally at least 8 hours, optionally at least 10 hours, optionally at least 12 hours, optionally at least 14 hours, optionally at least 16 hours, optionally at least 20 hours, optionally at least 24 hours, optionally at least 36 hours, optionally at least 48 hours, optionally at least 72 hours, optionally at least 96 hours.

Optionally, the further step of exposing the subject to a temperature of or above 21deg°C occurs for at least 48 hours. Further optionally, the further step of exposing the subject to a temperature of or above 21deg°C occurs for at least 48 hours prior to the step of exposing the subject to the stimulus at a second temperature different to the first temperature.

Optionally, the thermogenesis is non-shivering thermogenesis.

Optionally, the thermogenesis is induced in a muscle cell. Further optionally, the thermogenesis is induced in a muscle tissue.

Optionally, the thermogenesis is induced in a skeletal muscle cell. Further optionally, the thermogenesis is induced in a skeletal muscle tissue.

Optionally, the thermogenesis is non-shivering thermogenesis induced in a muscle cell. Further optionally, the thermogenesis is non-shivering thermogenesis induced in a muscle tissue.

Optionally, the thermogenesis is non-shivering thermogenesis induced in a skeletal muscle cell. Further optionally, the thermogenesis is non-shivering thermogenesis induced in a skeletal muscle tissue.

Optionally, the thermogenesis is induced in an adipose cell. Further optionally, the thermogenesis is induced in an adipose tissue.

Optionally, the thermogenesis is induced in a brown adipose cell. Further optionally, the thermogenesis is induced in a brown adipose tissue.

Optionally, the thermogenesis is non-shivering thermogenesis induced in an adipose cell. Further optionally, the thermogenesis is non-shivering thermogenesis induced in an adipose tissue.

Optionally, the thermogenesis is non-shivering thermogenesis induced in a brown adipose cell. Further optionally, the thermogenesis is non-shivering thermogenesis induced in a brown adipose tissue.

Preferably, the thermogenesis is non-shivering thermogenesis induced in a brown adipose cell. Further preferably, the thermogenesis is non-shivering thermogenesis induced in a brown adipose tissue.

Optionally, the adipose cell or tissue is selected from an interscapular, supraclavicular, suprarenal, pericardial, paraaortic, pancreatic, suprarenal, tracheal, paravertebral, mediastinal adipose cell or tissue, and combinations thereof.

Optionally, the subject is an animal. Further optionally, the subject is a mammal. Still further optionally, the subject is a human.

Preferably, the subject is a human.

Optionally, the method produces energy. Further optionally, the method produces heat.

Optionally, the method increases the temperature of the subject. Further optionally, the method increases the body temperature of the subject. Sill further optionally, the method increases the average/mean body temperature of the subject. Sill further optionally, the method increases the average/mean core body temperature of the subject.

Optionally or additionally, the method increases a rate of metabolism.

Optionally, the method increases a rate of glucose metabolism.

Optionally or additionally, the method increases a rate of lipid metabolism.

Optionally, the method increases oxygen consumption of the subject.

Optionally, the method increases energy expenditure of the subject.

Optionally, the method increases carbon dioxide production of the subject.

Optionally, the method increases locomotion of the subject.

Optionally, the method does not alter food consumption of the subject. Further optionally, the method does not alter the amount of food consumed by the subject.

Optionally, the method alters the expression of at least one thermogenic gene. Further optionally, the method increases the expression of at least one thermogenic gene.

Optionally, the at least one thermogenic gene is selected from Uncoupling Protein 1 (Ucp1), carnitine palmitoyltransferase 1a (Cpt1alpha), Carnitine-acylcarnitine translocase (Cact), Adipose triglyceride lipase (Atgl), Hormone-sensitive lipase (Hsl), and Protein c-Fos (c-Fos).

Optionally, the method alters the expression of Protein c-Fos (c-Fos). Further optionally, the method alters the expression of Protein c-Fos (c-Fos) in at least one of the cornu Ammonis 3 (CA3) and cornu Ammonis 1 (CA1) subregions of the hippocampus. Still further optionally, the method alters the expression of Protein c-Fos (c-Fos) in the cornu Ammonis 3 (CA3) and cornu Ammonis 1 (CA1) subregions of the hippocampus. Still further optionally, the method alters the expression of Protein c-Fos (c-Fos) in the lateral hypothalamic area (LHA) region of the brain.

### Brief description of the drawings

Embodiments of the invention will be described with reference to the appended non-limiting examples and the accompanying drawings in which:

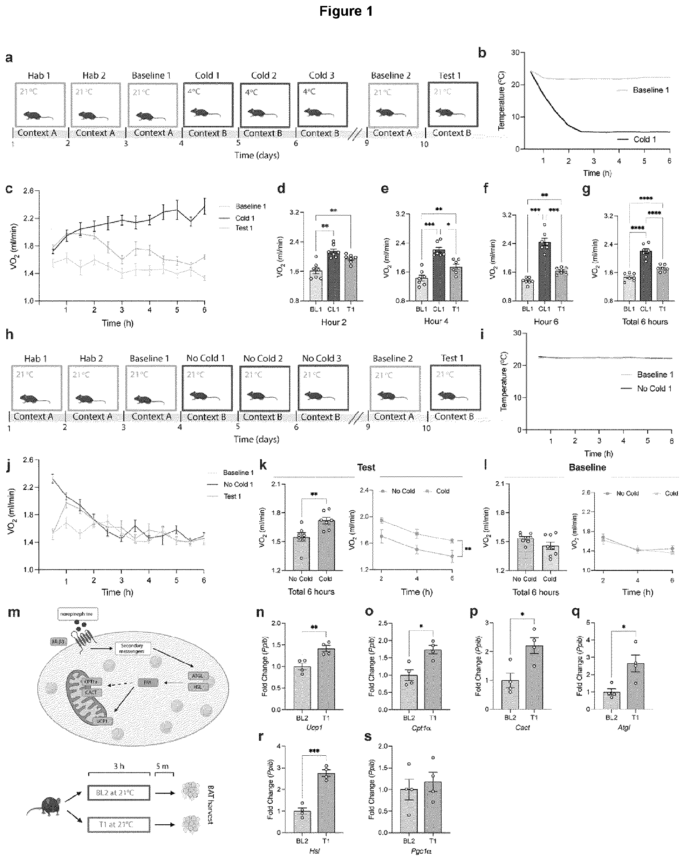

**Figure 1** illustrates contextual cold memories increase metabolic rate and thermogenic gene expression wherein (a) shows an experimental timeline, (b) shows environmental temperature during Baseline 1 (yellow) and Cold 1 (dark blue); (c) shows a time plot of oxygen consumption between mice during Baseline 1 (yellow), Test 1 (light blue) and Cold 1 (dark blue), with comparison of oxygen consumption between mice during Baseline 1 (yellow), Test 1 (light blue) and Cold 1 (dark blue) at (d) hour 2, (e) hour 4, (f) hour 6, and (g) total time averaged in metabolic cages; (h) shows an experimental control timeline; (i) shows environmental temperature during Baseline 1 (yellow) and No Cold 1 (dark blue); (j) shows a time plot of oxygen consumption between mice during Baseline 1 (yellow), Test 1 (light blue) and No Cold 1 (dark blue); (k) shows a comparison of oxygen consumption between the cold-paired (light blue) and no-cold paired (grey) cohorts on test day (6 hours averaged; left), and at hours 2, 4 and 6 (right); (l) shows a comparison of oxygen consumption between the cold-paired (yellow) and no-cold paired (grey) cohorts on Baseline 1 (6 hours averaged; left), and at hours 2, 4 and 6 (right); m shows a thermogenic pathway in BAT and experimental timeline with relative expression of lipolysis and thermogenesis genes (n) *Ucp1,* (o) *Cpt1α, (*p) *Cact, (*q) *Atgl,* (r) *Hsl* and (s) *Pgc1α* from the BAT of mice during BL2 (grey) compared to T1 (blue); wherein (c)-(g), (j)-(l), (n)-(s) data are shown as mean ± SEM, *n* = 4-8 mice per group; (d)-(g) show repeated measures ANOVA; (k)-(I) show an unpaired t-test and two-way repeated measures ANOVA; (n)-(s) show an unpaired t-test; wherein * p<0.05, ** p<0.01, *** p<0.001, **** p<0.0001, BL1, baseline 1; BL2, baseline 2; T1, Test 1; CL1, cold 1; CL2, cold 2; CL3, cold 3; VO₂, oxygen consumption; h, hour. BAT, brown adipose tissue; FFA, free fatty acid;

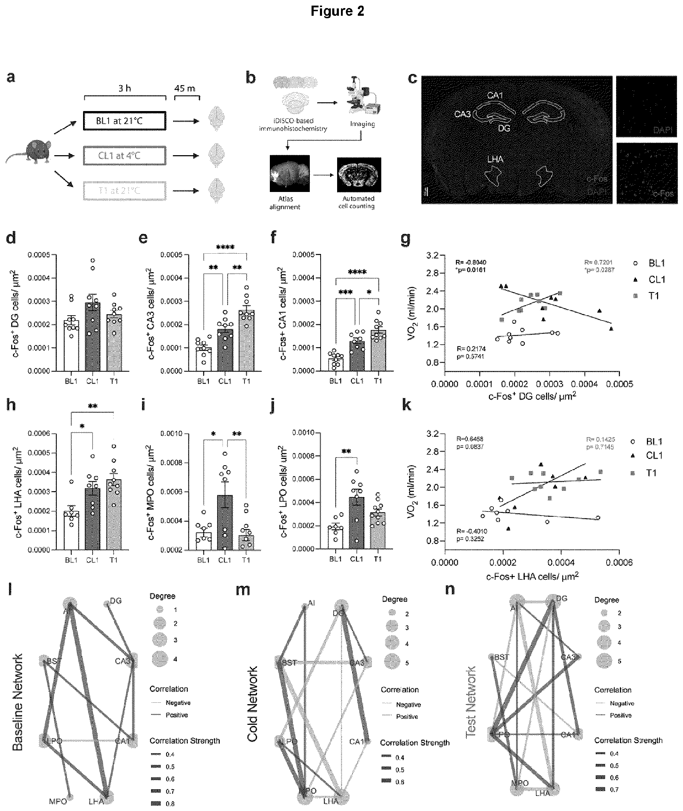

**Figure 2** illustrates contextual cold memories are encoded and retrieved in the hippocampus and hypothalamus, wherein (a) shows an experimental timeline for tissue collection; (b) shows a diagrammatic representation of the automated brain-wide c-Fos detection pipeline used to identify c-Fos⁺ cells in multiple brain regions simultaneously; (c) shows a representative image of hippocampal slice (left) with c-Fos⁺ cells (red; bottom-right) and DAPI⁺ cells (blue; top-right); with a comparison of c-Fos⁺ neurons normalized to area in the (d) DG, (e) CA3 and (f) CA1 between mice housed during BL1 (white), CL1 (dark blue) and T1 (light blue), (g) shows a correlation between c-Fos⁺ cells in the DG and oxygen consumption between mice housed during BL1 (white), CL1 (dark blue) and T1 (light blue), with a comparison of c-Fos⁺ neurons normalized to area in the (h) LHA, (i) MPO and (j) LPO between mice housed during BL1 (white), CL1 (dark blue) and T1 (light blue); (k) shows a correlation between c-Fos⁺ cells in the LHA and oxygen consumption between mice housed during BL1 (white), CL1 (dark blue) and T1 (light blue); (l) shows a correlation analysis of c-Fos⁺ cells between brain regions at (l) baseline, (m) cold exposure (CL1), and (n) cold recall (T1), with correlations with R>.5 displayed in red, correlations with R<.5 displayed in grey, wherein line thickness is proportional to the strength of the correlation (R value); wherein (d)-(f) and (h)-(j) data are shown as mean ± SEM, (d)-(n) *n =* 7-10 mice per group; (d)-(f) and (h)-(j) show One-way ANOVA; (g) and (k) show simple linear regression with slope comparison; wherein * p<0.05, ** p<0.01, *** p<0.001, **** p<0.0001, BL1 baseline 1; T1 Test 1; CL1 cold 1; DG dentate gyrus; CA3 cornu ammonis 3; CA1 cornu ammonis 1; VO₂ oxygen consumption; LHA lateral hypothalamic area; MPO medial preoptic area; LPO lateral preoptic area; BST bed nuclei of the stria terminalis; Al agranular insular area;

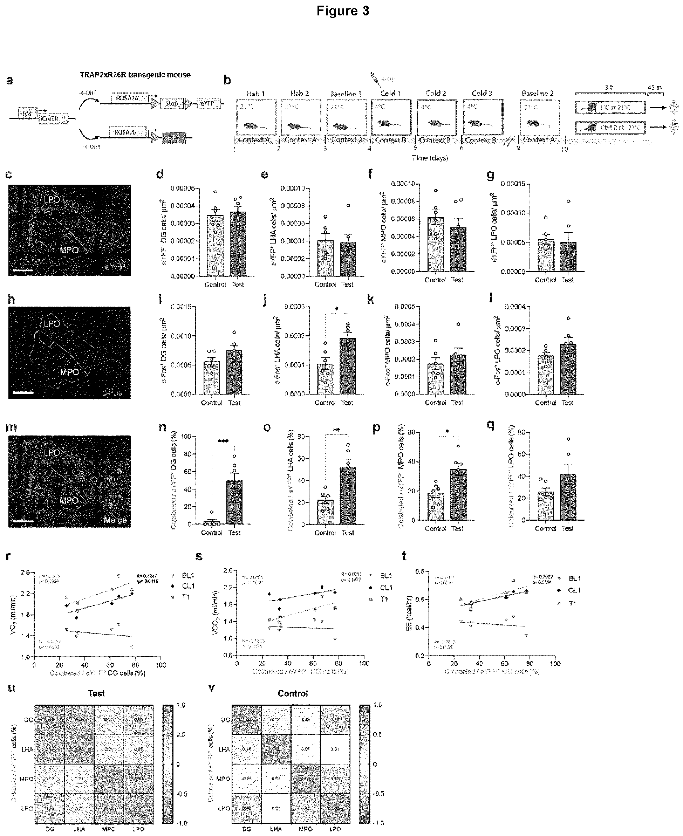

**Figure 3** illustrates putative contextual cold engrams are in the DG, LHA and MPO, wherein (a) shows a genetic strategy; (b) shows an experimental timeline; (c) shows a representative image of eYFP⁺ cells in green with a comparison of eYFP⁺ neurons normalized to area between the test cohort (dark blue) and control cohort (grey) in (d) the DG, (e) the LHA, (f) the MPO and (g) the LPO; (h) shows a representative image of c-Fos⁺ cells in red with a comparison of c-Fos⁺ neurons normalized to area between the test cohort (dark blue) and control cohort (grey) in (i) the DG, (j) the LHA, (k) the MPO and (l) the LPO; (m) shows a representative image of a eYFP⁺c-Fos⁺ co-labeled cell with a comparison of co-labeled neurons between the test cohort (dark blue) and control cohort (grey) in (n) the DG, (o) the LHA, (p) the MPO and (q) the LPO with a correlation between the percentage of co-labeled/eYFP⁺ cells in the DG and (r) oxygen consumption, (s) carbon dioxide production and (t) energy expenditure at T1 (orange), CL1 (black) and BL1 (grey); (u) shows a correlation of percentage of co-labeled/eYFP⁺ cells between regions during the recall of a cold memory on test day; (v) shows a correlation of percentage of co-labeled/eYFP⁺ cells of the control group; wherein (c)-(q) data are shown as mean ± SEM, (c)-(v) *n* = 6 mice per group; (d)-(g), (i)-(l), (n)-(q) show an unpaired t-test; (r)-(t) show a simple linear regression; (u)-(v) show Pearson's r correlation analysis; wherein * p<0.05, ** p<0.01, *** p<0.001, HC home cage; Ctx context; T1 test 1; h hour; m minutes; DG dentate gyrus; LHA lateral hypothalamic area; MPO medial preoptic area; LPO lateral preoptic area; VO₂ oxygen consumption; BL1 Baseline 1; CL1 Cold 1; T1 test 1; VCO₂ carbon dioxide production; EE energy expenditure;

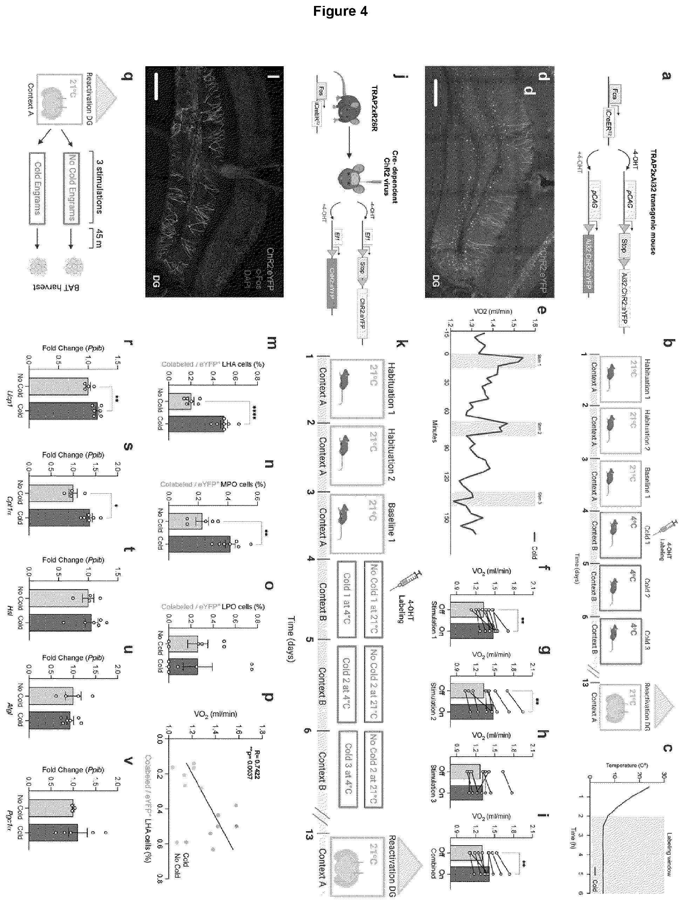

**Figure 4** illustrates artificial reactivation of cold-sensitive engrams in the DG increases metabolism and thermogenesis genes; wherein (a) shows a genetic strategy; (b) shows an experimental timeline; (c) shows environmental temperature during the labeling window; (d) shows a representative image of hippocampal slice with c-Fos⁺ cells (red) and eYFP⁺ cells (green); (e) shows a time plot of oxygen consumption during artificial reactivation of cold-sensitive engrams, wherein blue bars indicate the time the laser was turned on; (f), (g) and (h) show a comparison of oxygen consumption between the on (blue) and off (grey) epochs during the three stimulations; (i) shows a comparison of oxygen consumption between the on (blue) and off (grey) epochs of cold-sensitive cells during all stimulations combined; (j) shows a genetic strategy; (k) shows an experimental timeline; (l) shows a representative image of hippocampal slice with c-Fos⁺ cells (red) and eYFP⁺ cells (green) with a comparison of co-labeled neurons between the cold stimulated cohort (dark blue) and the no-cold stimulated control cohort (grey) in (m) the LHA, (n) the MPO and (o) the LPO; (p) shows a correlation between the percentage of co-labeled/eYFP⁺ cells in the LHA and oxygen consumption after cold stimulation (blue) and no-cold stimulation (grey); (q) shows an experimental timeline with relative expression of lipolysis and thermogenesis genes (r) *Ucp1,* (s) *Cpt1α (*t) *Hsl,* (u) *Atgl* and (v) *Pgc1α* from the BAT of mice with cold-sensitive stimulated cells (blue) and no-cold control stimulated cells (grey); wherein (e)-(i) data are shown as mean, *n* = 8-9. mice per group; wherein (m)-(p) and (r)-(v) data are shown as mean ± SEM, *n* = 5-7 mice per group; (e)-(i) show a paired *t*-test; (m)-(o) show an unpaired *t*-test; (p) shows a simple linear regression; (r)-(v) shows an unpaired *t*-test; wherein * p<0.05, ** p<0.01, *** p<0.001, **** p<0.0001; 4-OHT 4-hydroxytamoxifen; ChR2 Channelrhodopsin; DG dentate gyrus; VO₂ oxygen consumption; Stim 1 stimulation 1; Stim 2 stimulation 2; Stim 3 stimulation 3; LHA lateral hypothalamic area; MPO medial preoptic area; LPO lateral preoptic area; BAT, brown adipose tissue;

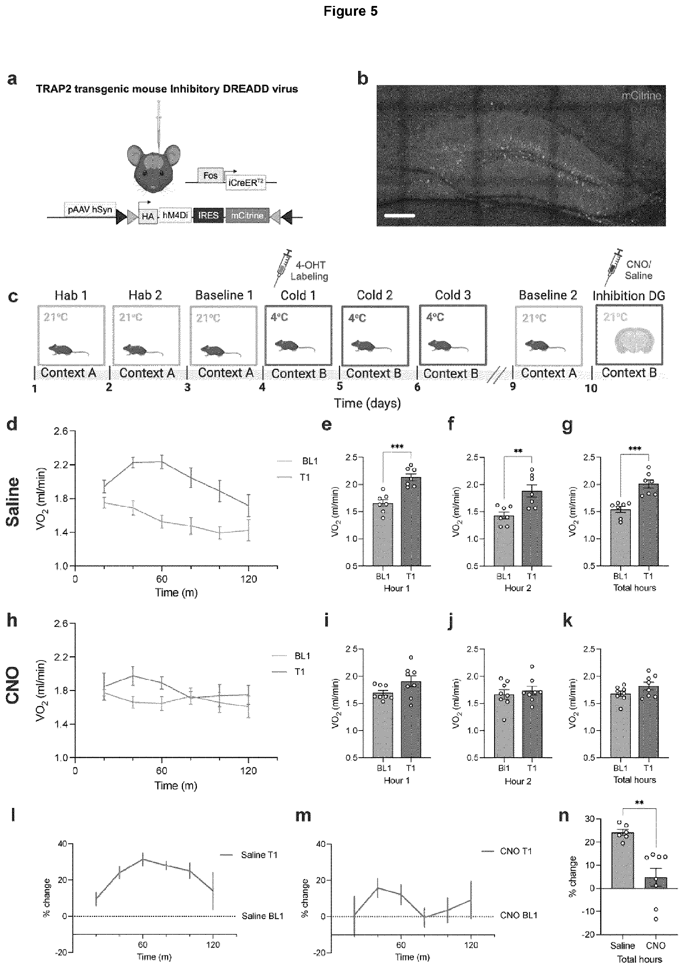

**Figure 5** illustrates inhibition of cold-sensitive engrams prevents the memory effects on oxygen consumption, wherein (a) shows a genetic strategy; (b) shows a representative image of hippocampal slice with c-Fos⁺ cells (red) and mCitrine⁺ cells (green); (c) shows an experimental timeline; (d) shows a time plot of oxygen consumption of saline injected mice during baseline 1 (grey) and test day 1 (teal); (e), (f) and (g) show a comparison of oxygen consumption of saline injected mice during baseline day 1 (grey) and test day 1 (teal) at hour one, hour two and total time averaged, respectively; (h) shows a time plot of oxygen consumption of CNO injected mice during baseline 1 (grey) and test day 1 (teal); (i), (j) and (k) show a comparison of oxygen consumption of CNO injected mice during baseline day 1 (grey) and test day 1 (teal) at hour one, hour two and total time averaged, respectively; (l) shows a time plot of the percentage change of oxygen consumption on test day 1 (teal) compared to baseline day 1 (black) in saline injected mice; (m) shows a time plot of the percentage change of oxygen consumption on test day 1 (teal) compared to baseline day 1 (black) in CNO injected mice; (n) shows a comparison of the percentage change of oxygen consumption between saline (grey) and CNO (teal) injected mice on test day 1, wherein data are shown as mean ± SEM, (d)-(n) *n* = 7-8 mice per group; (e)-(g) and (i)-(k) show a paired *t*-test; (n) shows an unpaired *t*-test, wherein ** p<0.01, *** p<0.001, 4-OHT 4-hydroxytamoxifen; VO2 oxygen consumption; T1 test 1; BL1 baseline 1; m minutes; CNO clozapine N-oxide;

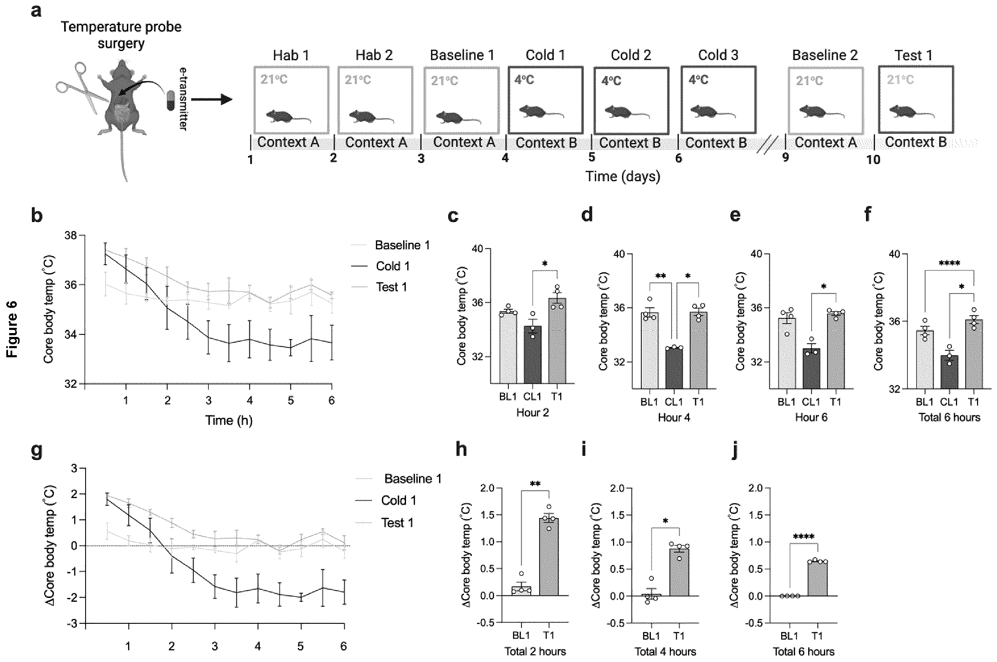

**Figure 6** illustrates retrieval of a contextual cold memory increases core body temperature, wherein (a) shows a diagrammatic representation of an experimental timeline; (b) shows a time plot of core body temperature between mice during Baseline 1 (21deg°C; yellow), Test 1 (21deg°C; light blue) and Cold 1 (4deg°C; dark blue) with a comparison of core body temperature between mice during Baseline 1 (21deg°C; yellow), Test 1 (21deg°C; light blue) and Cold 1 (4deg°C; dark blue) at (c) hour 2, (d) hour 4, (e) hour 6 and (f) total time averaged in metabolic cages; (g) shows a time plot of the change in core body temperature between mice during Baseline 1 (21deg°C; yellow), Test 1 (21deg°C; light blue) and Cold 1 (4deg°C; dark blue) normalized to the average core body temperature on BL1 with a time plot of the change in core body temperature between mice during Baseline 1 (21deg°C; yellow) and Test 1 (21deg°C; light blue) during (h) the total 2 hours, (i) the total 4 hours and (j) total time averaged in metabolic cages; wherein (b)-(j) data are shown as mean ± SEM, *n* = 4 mice per group; (c)-(f) show a repeated measures ANOVA; (h)-(j) show a paired t-test; wherein * p<0.05, ** p<0.01, **** p<0.0001; h hour; BL1 baseline 1; CL1 Cold 1; T1 Test 1;

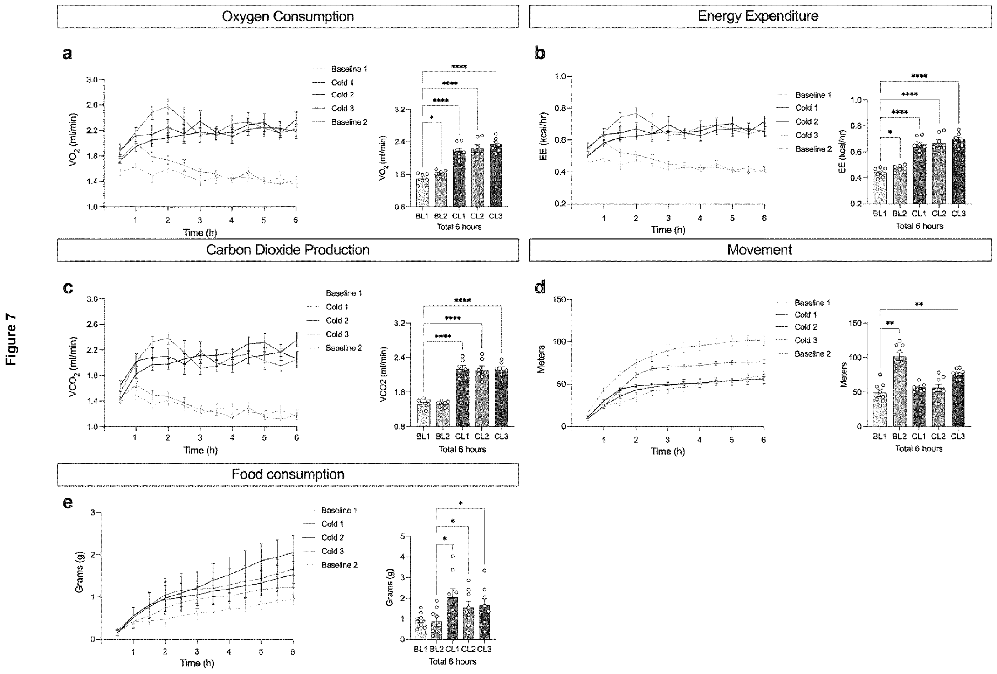

**Figure 7** illustrates cold exposure increases metabolic rate and alters behavior, wherein (a) shows a time plot of oxygen consumption between mice during Baseline 1 (21deg°C; yellow), Baseline 2 (21deg°C; orange), Cold 1 (4deg°C; dark blue), Cold 2 (4deg°C; blue), and Cold 3 (4deg°C; light blue; left), with comparisons of total time averaged in metabolic cages (right); (b) shows a time plot of energy expenditure between mice during Baseline 1 (21deg°C; yellow), Baseline 2 (21deg°C; orange), Cold 1 (4deg°C; dark blue), Cold 2 (4deg°C; blue), and Cold 3 (4deg°C; light blue; left), with comparisons of total time averaged in metabolic cages (right); (c) shows a time plot of carbon dioxide production between mice during Baseline 1 (21deg°C; yellow), Baseline 2 (21deg°C; orange), Cold 1 (4deg°C; dark blue), Cold 2 (4deg°C; blue), and Cold 3 (4deg°C; light blue; left), with comparisons of total time averaged in metabolic cages (right); (d) shows a time plot of movement between mice during Baseline 1 (21deg°C; yellow), Baseline 2 (21deg°C; orange), Cold 1 (4deg°C; dark blue), Cold 2 (4deg°C; blue), and Cold 3 (4deg°C; light blue; left), with comparisons of total time averaged in metabolic cages (right); (e) shows a time plot of food consumption between mice during Baseline 1 (21deg°C; yellow), Baseline 2 (21deg°C; orange), Cold 1 (4deg°C; dark blue), Cold 2 (4deg°C; blue), and Cold 3 (4deg°C; light blue; left), with comparisons of total time averaged in metabolic cages (right); wherein (a)-(e) data are shown as mean ± SEM, *n* = 7-8 mice per group; (a)-(e) show a repeated measures ANOVA; wherein * p<0.05, ** p<0.01, *** p<0.001, **** p<0.0001; BL1 baseline 1; BL2 baseline 2; CL1 cold 1; CL2 cold 2; CL3 cold 3; VO₂ oxygen consumption; VCO₂ carbon dioxide emission; h hour; g grams;

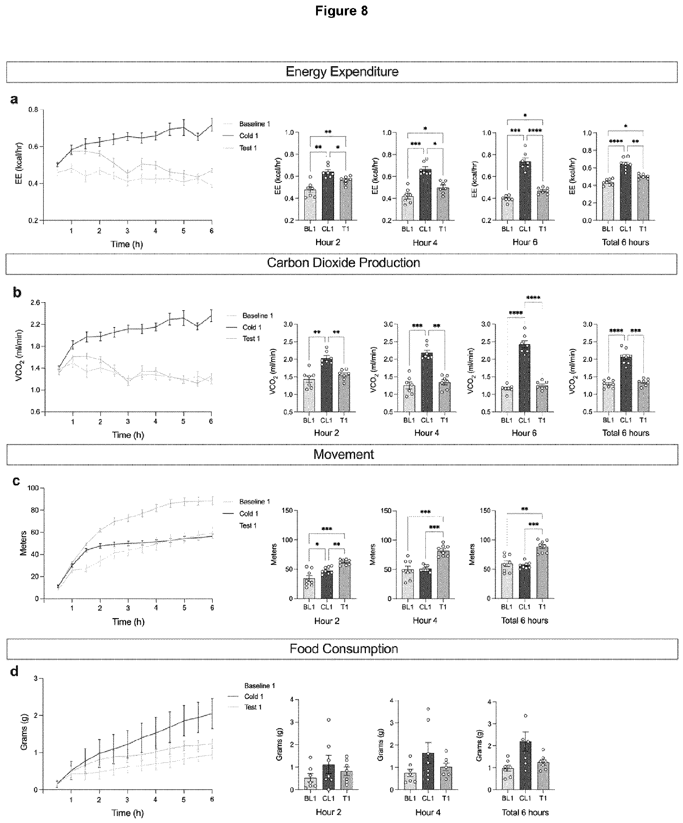

**Figure 8** illustrates a memory of a cold-paired context is sufficient to increase metabolic rate and alter behavior; wherein (a) shows a time plot of energy expenditure between mice during Baseline 1 (21deg°C; yellow), Test 1 (21deg°C; light blue) and Cold 1 (4deg°C; dark blue; left), with comparisons at hour 2, hour 4, hour 6 and total time averaged in metabolic cages (right); (b) shows a time plot of carbon dioxide production between mice during Baseline 1 (21deg°C; yellow), Test 1 (21deg°C; light blue) and Cold 1 (4deg°C; dark blue; left), with comparisons at hour 2, hour 4, hour 6 and total time averaged in metabolic cages (right); (c) shows a time plot of movement between mice during Baseline 1 (21deg°C; yellow), Test 1 (21deg°C; light blue) and Cold 1 (4deg°C; dark blue; left), with comparisons at hour 2, hour 4 and total time averaged in metabolic cages (right); (d) shows a time plot of food consumption between mice during Baseline 1 (21deg°C; yellow), Test 1 (21deg°C; light blue) and Cold 1 (4deg°C; dark blue; left), with comparisons at hour 2, hour 4 and total time averaged in metabolic cages (right); wherein (a)-(d) data are shown as mean ± SEM, *n* = 7-8 mice per group; (a)-(d) show a repeated measures ANOVA; wherein * p<0.05, ** p<0.01, *** p<0.001, **** p<0.0001. BL1 baseline 1; T1 Test 1; CL1 cold 1; VCO₂ carbon dioxide emission; EE energy expenditure; h hour;

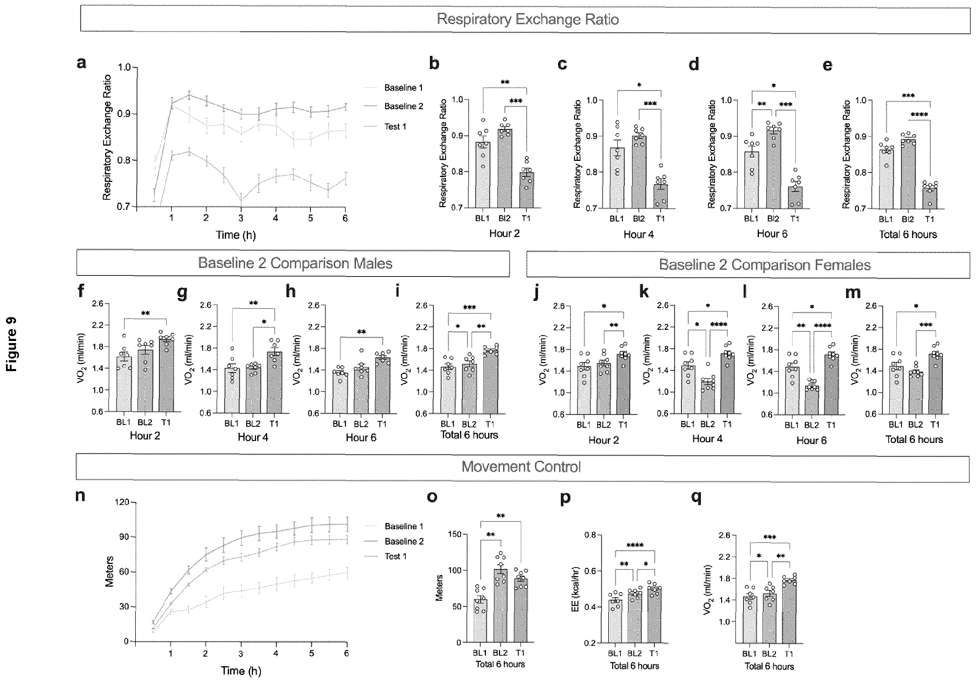

**Figure 9** illustrates increases in metabolic rate on T1 compared to BL1 and BL2 is not due to activity; wherein (a) shows a time plot of the respiratory exchange ratio between mice during BL1 (21deg°C; yellow), BL2 (21deg°C; orange) and T1 (21deg°C; light blue) with a comparison of the respiratory exchange ratio between mice during BL1 (21deg°C; yellow), BL2 (21deg°C; orange) and T1 (21deg°C; light blue) at (b) hour 2, (c) hour 4, (d) hour 6, and (e) total time averaged in metabolic cages with a comparison of oxygen consumption between male mice during BL1 (21deg°C; yellow), BL2 (21deg°C; orange) and T1 (21deg°C; light blue) at (f) hour 2, (g) hour 4, (h) hour 6 and (i) total time averaged in metabolic cages with a comparison of oxygen consumption between female mice during BL1 (21deg°C; yellow), BL2 (21deg°C; orange) and T1 (21deg°C; light blue) at (j) hour 2, (k) hour 4, (l) hour 6, and (m) total time averaged in metabolic cages; (n) shows a time plot of movement between mice during BL1 (21deg°C; yellow), BL2 (21deg°C; orange) and T1 (21deg°C; light blue); (o) shows a comparison of movement between mice during BL1 (21deg°C; yellow), BL2 (21deg°C; orange) and T1 (21deg°C; light blue) for the total time averaged in metabolic cages; (p) shows a comparison of energy expenditure between mice during BL1 (21deg°C; yellow), BL2 (21deg°C; orange) and T1 (21deg°C; light blue) for the total time averaged in metabolic cages; (q) shows a comparison of oxygen consumption between mice during BL1 (21deg°C; yellow), BL2 (21deg°C; orange) and T1 (21deg°C; light blue) for the total time averaged in metabolic cages, wherein (a)-(q) data are shown as mean ± SEM, *n* = 7-8 mice per group; (b)-(m) and (o)-(q) show a repeated measures ANOVA, wherein * p<0.05, ** p<0.01, *** p<0.001, **** p<0.0001; BL1 baseline 1; BL2 baseline 2; T1 Test 1; EE energy expenditure; VO₂ oxygen consumption; h hour;

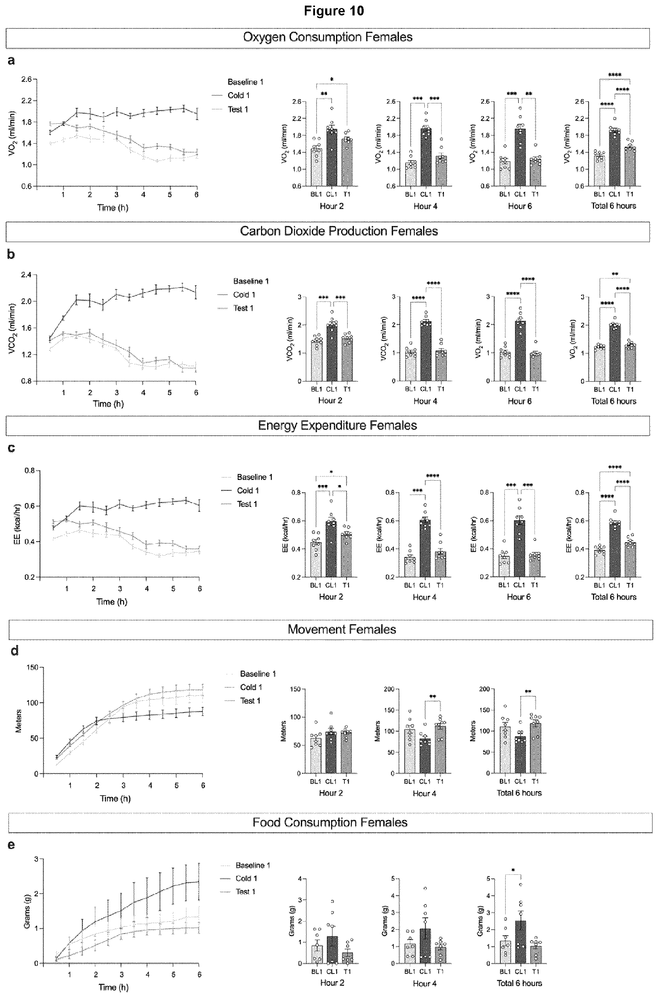

**Figure 10** illustrates a memory of a cold-paired context is sufficient to increase metabolic rate and alter behavior in females; wherein (a) shows a time plot of oxygen consumption between female mice during Baseline 1 (21deg°C; yellow), Test 1 (21deg°C; purple) and Cold 1 (4deg°C; dark blue; left), with comparisons at hour 2, hour 4, hour 6 and total time averaged in metabolic cages (right); (b) shows a time plot of carbon dioxide production between female mice during Baseline 1 (21deg°C; yellow), Test 1 (21deg°C; purple) and Cold 1 (4deg°C; dark blue; left), with comparisons at hour 2, hour 4, hour 6 and total time averaged in metabolic cages (right); (c) shows a time plot of energy expenditure between female mice during Baseline 1 (21deg°C; yellow), Test 1 (21deg°C; purple) and Cold 1 (4deg°C; dark blue; left), with comparisons at hour 2, hour 4, hour 6 and total time averaged in metabolic cages (right); (d) shows a time plot of movement between female mice during Baseline 1 (21deg°C; yellow), Test 1 (21deg°C; purple) and Cold 1 (4deg°C; dark blue; left), with comparisons at hour 2, hour 4 and total time averaged in metabolic cages (right); (e) shows a time plot of food consumption between female mice during Baseline 1 (21deg°C; yellow), Test 1 (21deg°C; light blue) and Cold 1 (4deg°C; dark blue; left), with comparisons at hour 2, hour 4 and total time averaged in metabolic cages (right), wherein (a)-(e) data are shown as mean ± SEM, *n* = 7-8 mice per group; (a)-€ shows a repeated measures ANOVA, wherein * p<0.05, ** p<0.01, *** p<0.001, **** p<0.0001; BL1 baseline 1; T1 Test 1; CL1 cold 1; VO₂ oxygen consumption; VCO₂ carbon dioxide emission; EE energy expenditure; h hour;

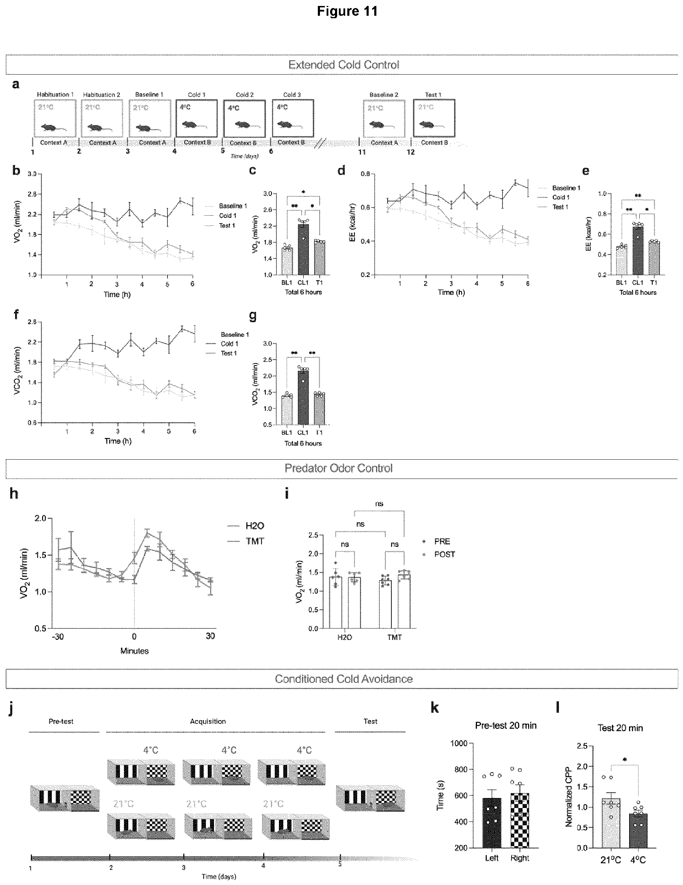

**Figure 11** illustrates a cold memory is maintained after a prolonged break, is not due to stress, and causes avoidance behavior; wherein (a) shows a diagrammatic representation of an experimental timeline for Pavlovian conditioning to a cold-paired context with an extended (4 day) break; (b) shows a time plot of oxygen consumption between mice during BL1 (21deg°C; yellow), T1 (21deg°C; light blue) and CL1 (4deg°C; dark blue) following an extended (4 day) break, with (c) showing comparisons of total time averaged in metabolic cages (right); (d) shows a time plot of energy expenditure between mice during BL1 (21deg°C; yellow), T1 (21deg°C; light blue) and CL1 (4deg°C; dark blue) following an extended (4 day) break, with € showing comparisons of total time averaged in metabolic cages (right); (f) shows a time plot of carbon dioxide production between mice during BL1 (21deg°C; yellow), T1 (21deg°C; light blue) and CL1 (4deg°C; dark blue) following an extended (4 day) break, with (g) showing comparisons of total time averaged in metabolic cages (right); (h) shows a time plot of oxygen consumption between mice exposed to TMT (orange) or control mice exposed to H₂O (grey); (i) shows a comparison of pre (grey) and post (orange) odor exposure between mice exposed to TMT and control mice exposed to H₂O; (j) shows a diagrammatic representation of experimental timeline for Pavlovian conditioning to the cold-paired side, or no cold paired side, of a CPP apparatus; (k) shows time spent in each chamber of the CPP apparatus during pre-test; and (I) shows place preference for the no cold paired side (yellow) of the CPP apparatus compared to the cold-paired side (blue), time spent in either the cold-paired side, or no cold paired side was divided by the time spent in the same chamber during pre-test (Test/Pre-test); wherein (b)-(l) and (k),(I) data are shown as mean ± SEM, *n* = 5-7 mice per group; (c), (e) and (g) show a repeated measures ANOVA; (i) shows a two-way ANOVA; (k) and (l) show an unpaired t-test, wherein * p<0.05, ** p<0.01; BL1 baseline 1; T1 Test 1; CL1 cold 1; VO₂ oxygen consumption; VCO₂ carbon dioxide emission; h hour; TMT trimethylthiazoline; s seconds; CPP conditioned place preference;

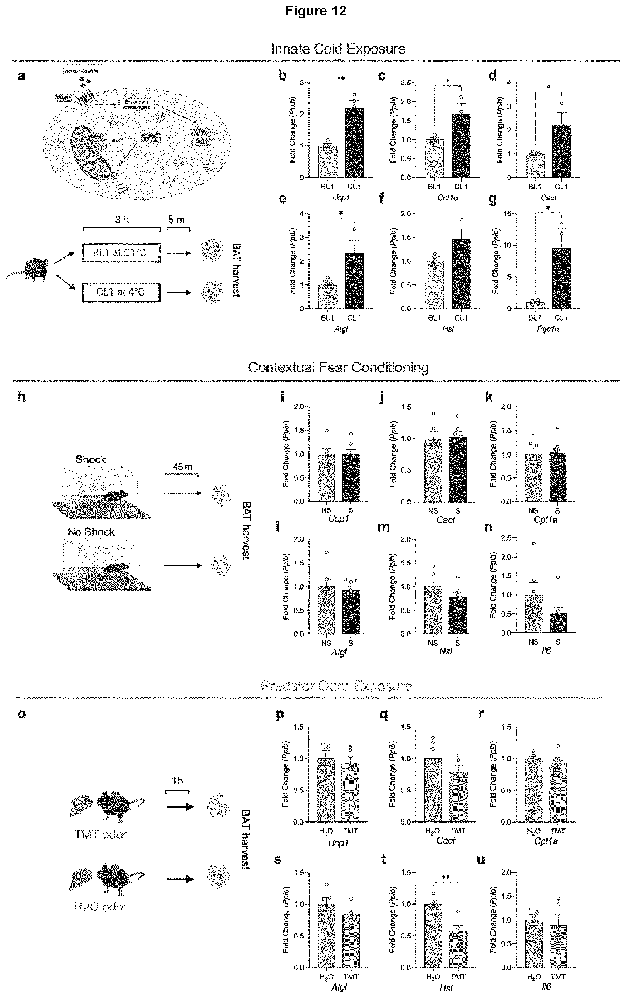

**Figure 12** illustrates innate cold exposure, but not stress, increases lipolytic and thermogenic gene expression in BAT, wherein (a) shows a diagrammatic representation of the thermogenic pathway in BAT (above), with experimental timeline for tissue collection (below) with relative expression of lipolysis and thermogenesis genes (b) *Ucp1,* (c) *Cpt1α, (*d) *Cact, (*e) *Atgl,* (f) *Hsl* and (g) *Pgc1α* from the BAT of mice housed during BL1 (grey) compared cold exposed mice (CL1; blue); (h) shows a diagrammatic representation of experimental timeline for tissue collection with relative expression of lipolysis and thermogenesis genes (i) *Ucp1, (*j) *Cact, (*k) *Cpt1α, (*l) *Atgl,* (m) *Hsl* and (n) *II6* from the BAT of mice that received foot shocks compared to no shock controls; *n* = 6-7 mice per group; (o) shows a diagrammatic representation of experimental timeline for tissue collection with relative expression of lipolysis and thermogenesis genes (p) *Ucp1, (*q) *Cact, (*r) *Cpt1α, (*s) *Atgl,* (t) *Hsl* and (u) *II6* from the BAT of mice that were exposed to TMT and H2O exposed controls; wherein (b)-(g), (i)-(n) (p)-(u) data are shown as mean ± SEM, *n* = 3-7 mice per group; (b)-(g), (i)-(n) and (p)-(u) show an unpaired t-test, wherein * p<0.05, ** p<0.01; BAT brown adipose tissue; FFA free fatty acid; BL1 baseline 1; CL1 cold 1; m minutes; NS no shock; S shocked; TMT trimethylthiazoline; h hour;

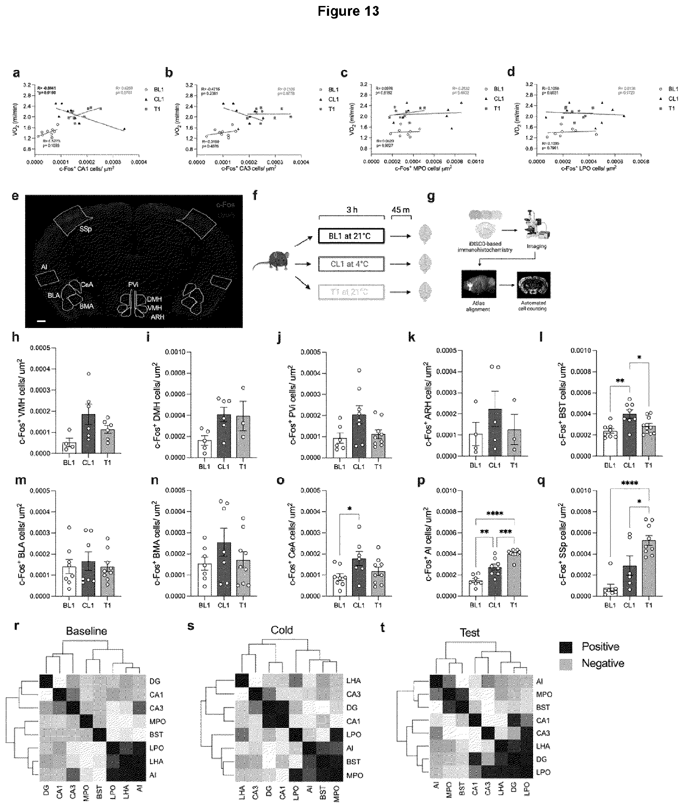

**Figure 13** illustrates retrieval of a cold memory does not correlate metabolism with c-Fos⁺ in CA3, MPO or LPO, or increase neural activity in every region, with a correlation between oxygen consumption and c-Fos⁺ cells in (a) CA1, (b) CA3, (c) MPO, and (d) LPO between mice during BL1 (white), CL1 (dark blue) and T1 (light blue); (e) shows a representative image of hippocampal slice with c-Fos⁺ cells (red) and DAPI⁺ cells (blue); (f) shows an experimental timeline for tissue collection; (g) shows a diagrammatic representation of the automated brain-wide c-Fos detection pipeline used to identify c-Fos⁺ cells in multiple brain regions simultaneously, with a comparison of c-Fos⁺ neurons normalized to area in the (h) VMH, (i) DMH, (j) PVi, (k) ARH, (l) BST, (m) BLA, (n) BMA, (o) CeA, (p) Al and (q) SSp between mice housed during BL1 (white), CL1 (dark blue) and T1 (light blue), with a correlation matrix of c-Fos⁺ cells normalized to area during (r) baseline day (BL1), (s) cold training day (CL1) and (t) the recall of a contextual cold memory on test day, with positive (red) and negative (grey) correlations; wherein (h)-(t) data are shown as mean ± SEM, (a)-(d) and (h)-(t) *n* = 3-9 mice per group; (a)-(d) show a simple linear regression with slope comparison, (h)-(q) show a one-way ANOVA; wherein * p<0.05, ** p<0.01, *** p<0.001, **** p<0.0001; BL1 baseline 1; T1 Test 1; CL1 cold 1; CA3 cornu ammonis 3; CA1 cornu ammonis 1; VO₂ oxygen consumption; MPO medial preoptic area; LPO lateral preoptic area; VMH ventromedial hypothalamic nucleus; DMH dorsomedial nucleus of the hypothalamus; Al agranular insular area; SSp primary somatosensory area; PVi periventricular hypothalamic nucleus-intermediate part; ARH arcuate hypothalamic nucleus; BST bed nuclei of the stria terminalis; BLA basolateral amygdalar nucleus; BMA basomedial amygdalar nucleus; CeA central amygdalar nucleus; DG dentate gyrus; LHA lateral hypothalamic area;

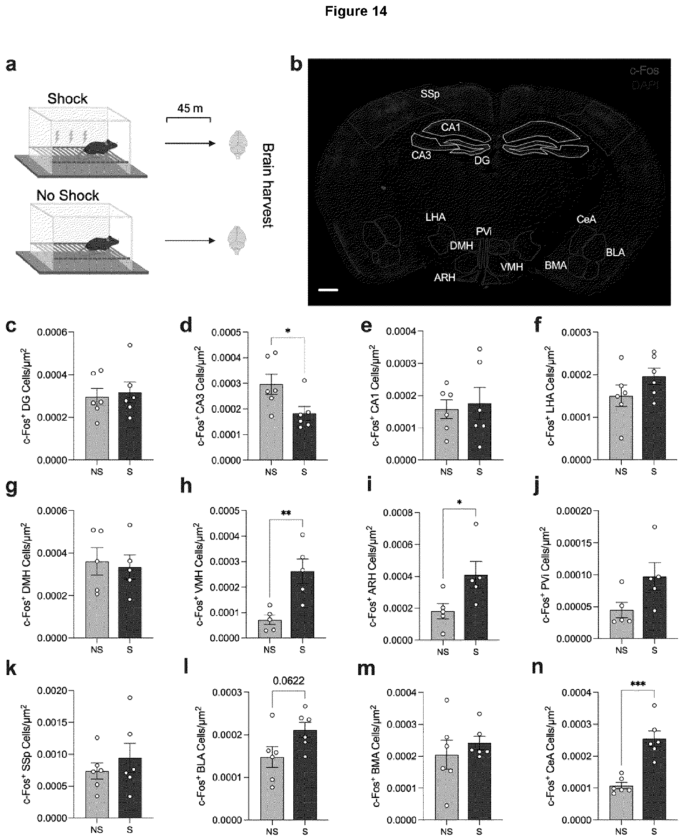

**Figure 14** illustrates distinct stress-related stimuli activate divergent brain regions, wherein (a) shows a diagrammatic representation of experimental timeline for tissue collection; (b) shows a representative image of hippocampal slice with c-Fos⁺ cells (red) and DAPI⁺ cells (blue) with a comparison of c-Fos⁺ neurons normalized to area in the (c) DG, (d) CA3, (e) CA1, (f) LHA, (g) DMH, (h) VMH, (i) ARH, (j) PVi, (k) SSp, (l) BLA, (m) BMA, and (n) CeA of mice that received foot shocks (red) compared to no shock controls (grey), wherein data are shown as mean ± SEM, (c)-(n) *n* = 5-6 mice per group; (c)-(n) show an unpaired *t*-test, wherein * p<0.05, ** p<0.01, *** p<0.001, S shocked; NS no shock; DG dentate gyrus; CA3 cornu ammonis 3; CA1 cornu ammonis 1; LHA lateral hypothalamic area; DMH dorsomedial nucleus of the hypothalamus; VMH ventromedial hypothalamic nucleus; ARH arcuate hypothalamic nucleus; PVi periventricular hypothalamic nucleus-intermediate part; SSp primary somatosensory area; BLA basolateral amygdalar nucleus; BMA basomedial amygdalar nucleus; CeA central amygdalar nucleus;

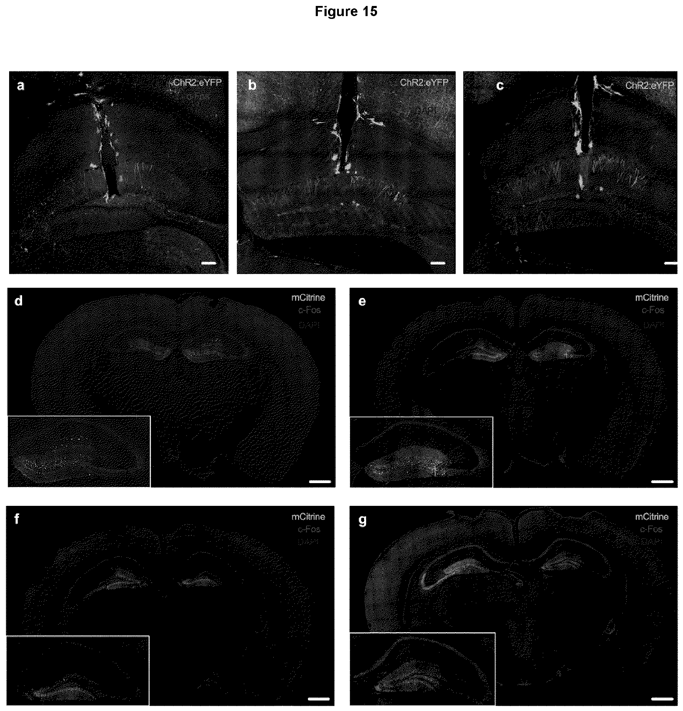

**Figure 15** illustrates a representative images of optogenetic and chemogenetic experiments, wherein (a)-(c) show a representative images of hippocampal slices with c-Fos⁺ cells (red), ChR2⁺ cells (green), DAPI⁺ cells (blue) and optogenetic implant tracts; and (d)-(g) show representative images of hippocampal slices with c-Fos⁺ cells (red), mCitrine⁺ cells (green) and DAPI⁺ cells (blue) of chemogenetic experiments;

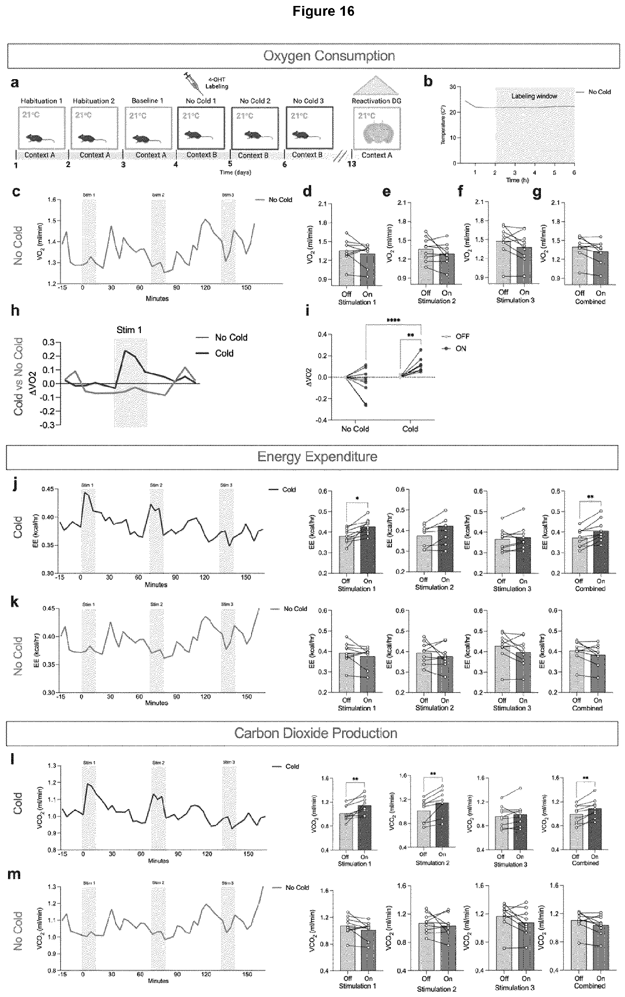

**Figure 16** illustrates artificial reactivation of cold-sensitive memory engrams in the DG increases metabolic rate, wherein (a) shows an experimental timeline to label and reactivate no cold engrams; (b) shows environmental temperature during the labelling window on no cold day 1; (c) shows a time plot of oxygen consumption during artificial reactivation of no cold control engrams (Blue bars indicate laser activation); (d), (e) and (f) show a comparison of oxygen consumption between the on (dark grey) and off (light grey) epochs during the three stimulations of no cold control cells, with (g) showing all stimulations combined; (h) shows a change of oxygen consumption before, during and after stimulating cold-sensitive engram cells (blue) and no cold control cells (grey) normalized to the initial 20 minutes of recording (i) shows a comparison of the change in oxygen consumption during on (blue) and off (grey) epochs between cold-sensitive stimulated cells and no cold control stimulated cells, with a time plot of energy expenditure during artificial reactivation of (j), cold-sensitive engrams (left) and (k) no cold control cells (left), with comparison of oxygen consumption between the on (blue) and off (grey) epochs during the three stimulations of cold-sensitive cells (right), with a time plot of carbon dioxide production during artificial reactivation of (I), cold-sensitive engrams (left) and (m), no cold control cells (left), with comparison of oxygen consumption between the on (blue) and off (grey) epochs during the three stimulations of cold-sensitive cells (right), wherein blue bars indicate the time the laser was turned on during the 3 stimulation periods; wherein (d)-(g) and (i)-(m) data are shown as mean, (c)-(m) *n* = 8-9 mice per group; (d)-(g) and (j)-(m) show an unpaired t-test, (i) shows a two-way ANOVA, wherein * p<0.05, ** p<0.01, **** p<0.0001; VCO₂ carbon dioxide production; Stim 1 stimulation 1; Stim 2 stimulation 2; Stim 3 stimulation 3;

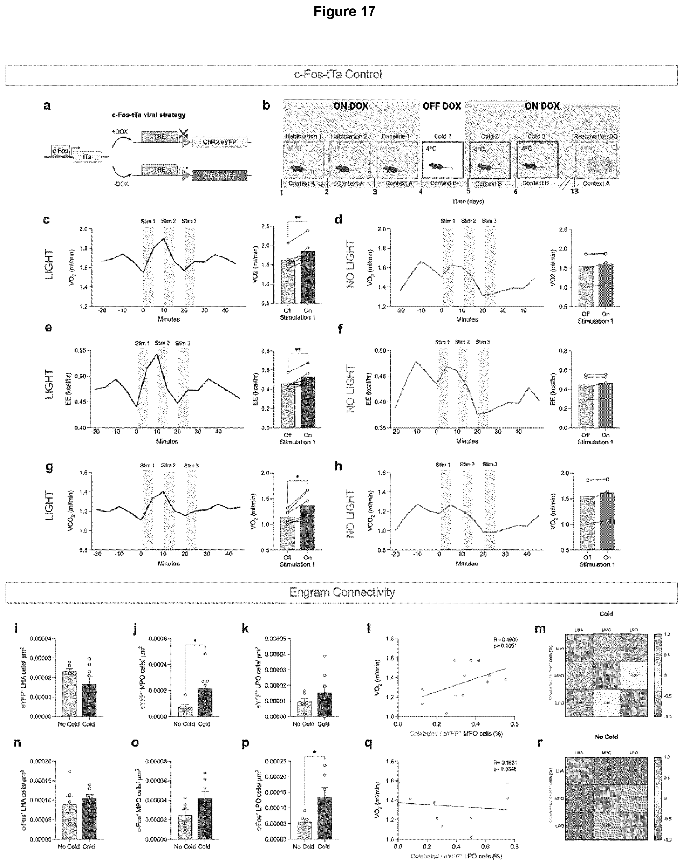

**Figure 17** illustrates artificial reactivation of cold-sensitive memory engrams in the DG increases metabolic rate in c-Fos tTA mice and engram activity in the hypothalamus, wherein (a) shows a diagrammatic representation of the c-Fos tTA transgenic labeling system for ChR2::GFP expression and (b) shows a timeline to label cold-sensitive engrams with c-Fos tTA mice, with a time plot of (c) oxygen consumption, (e) energy expenditure, and (g) carbon dioxide production during artificial reactivation of cold-sensitive engrams (left), with comparison of on (blue) and off (grey) epochs during the first stimulation of cold-sensitive cells (right), with a time plot of (d) oxygen consumption, (f) energy expenditure and (h) carbon dioxide production during artificial reactivation of no cold control cells (left), with comparison of on (blue) and off (grey) epochs during the first stimulation of no cold control cells (right), with a comparison of eYFP⁺ neurons normalized to area between the cold stimulated (dark blue) and no-cold stimulated (grey) cohorts in (i) the LHA, (j) the MPO and (k) the LPO, with a comparison of c-Fos⁺ neurons normalized to area between the cold stimulated (dark blue) and no-cold stimulated (grey) cohorts in (n) the LHA, (o) the MPO and (p) the LPO, with a correlation between the percentage of colabeled/ eYFP⁺ cells in (i) the MPO and (q) the LPO with oxygen consumption after cold stimulation (blue) and no-cold stimulation (grey), with a correlation of percentage of co-labeled/eYFP⁺ cells between regions after optogenetic stimulation of (m) cold-sensitive engrams and (r) no-cold engrams, wherein blue bars indicate the time the laser was turned on during the 3 stimulation periods, wherein (a)-(h) data are show as mean and (i)-(k) and (n)-(p) as mean ± SEM, *n* = 6-7 mice per group, (c)-(h) and (i)-(p) show an unpaired t-test, (l) and (q) show a simple linear regression, wherein * p<0.05, ** p<0.01; ChR2 channelrhodopsin-2; GFP green fluorescent protein; DOX doxycycline; VO₂ oxygen consumption; VCO₂ carbon dioxide production; Stim 1 stimulation 1; Stim 2 stimulation 2; Stim 3 stimulation 3; LHA lateral hypothalamic area; MPO medial preoptic area; LPO lateral preoptic area; and

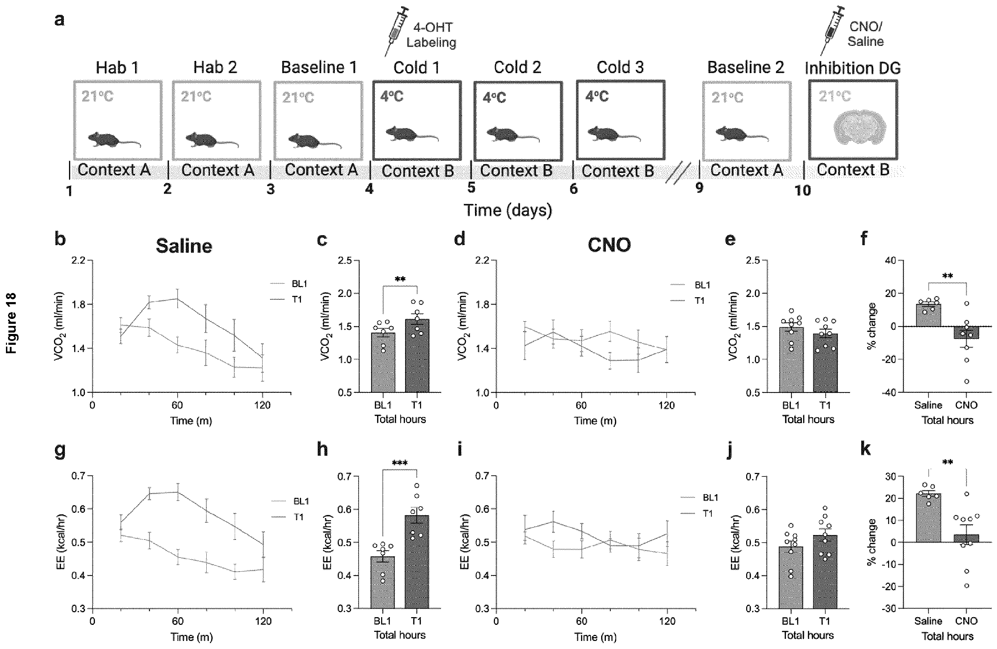

**Figure 18** **illustrates inhibition of cold-sensitive engrams prevents the memory associated increases in metabolic rates, wherein (a) illustrates a d**iagrammatic representation of an experimental timeline to label and chemogenetically inhibit contextual cold memory engrams; **(b) shows a** t**ime** plot of carbon dioxide production of saline injected mice during baseline 1 (grey) and test day 1 (teal), with **(c)** the total time averaged, **(d) a t**ime plot of carbon dioxide production of CNO injected mice during baseline 1 (grey) and test day 1 (teal) with **(e)** the total time averaged, **(f) a c**omparison of the percentage change of carbon dioxide production between saline (grey) and CNO (teal) injected mice on test day 1; **(g) shows a t**ime plot of energy expenditure of saline injected mice during baseline 1 (grey) and test day 1 (teal) with **(h)** the total time averaged, **(i) a t**ime plot of energy expenditure of CNO injected mice during baseline 1 (grey) and test day 1 (teal) with (**j)** the total time averaged; **(k) shows a c**omparison of the percentage change of energy expenditure between saline (grey) and CNO (teal) injected mice on test day 1, wherein data are shown as mean ± SEM **(b)-(k)** *n* = 7-8 mice per group; **(c), (e), (h), (j) show a** paired *t*-test, **(f) and (k) show an** unpaired *t*-test, wherein ** p<0.01, *** p<0.001; 4-OHT 4-hydroxytamoxifen; VCO2 carbon dioxide emission; EE energy expenditure; T1 test 1; BL1 baseline 1; m minutes; CNO clozapine N-oxide.

### Examples

### Materials and methods

### Mice

All wild-type experiments were performed with C57BL/6J mice. All engram-labeling experiments were performed with **c-fos-tTa** (Liu, X., Ramirez, S., Pang, P. T., Puryear, C. B., Govindarajan, A., Deisseroth, K. & Tonegawa, S. Optogenetic stimulation of a hippocampal engram activates fear memory recall. Nat. 2012 4847394 484, 381-385 (2012); Reijmers, L. G., Perkins, B. L., Matsuo, N. & Mayford, M. Localization of a Stable Neural Correlate of Associative Memory. Science 317, 1230-1233 (2007)), **TRAP2 x Ai32** (DeNardo, L. A., Liu, C. D., Allen, W. E., Adams, E. L., Friedmann, D., Fu, L., Guenthner, C. J., Tessier-Lavigne, M. & Luo, L. Temporal Evolution of Cortical Ensembles Promoting Remote Memory Retrieval. Nat. Neurosci. 22, 460 (2019); Madisen, L., Mao, T., Koch, H., Zhuo, J., Berenyi, A., Fujisawa, S., Hsu, Y.-W. A., Garcia, A. J., Gu, X., Zanella, S., Kidney, J., Gu, H., Mao, Y., Hooks, B. M., Boyden, E. S., Buzsáki, G., Ramirez, J. M., Jones, A. R., Svoboda, K., Han, X., Turner, E. E. & Zeng, H. A toolbox of Cre-dependent optogenetic transgenic mice for light-induced activation and silencing. Nat. Neurosci. 15, 793-802 (2012) and **TRAP2 x R26R** (Denny, C. A., Kheirbek, M. A., Alba, E. L., Tanaka, K. F., Brachman, R. A., Laughman, K. B., Tomm, N. K., Turi, G. F., Losonczy, A. & Hen, R. Hippocampal Memory Traces Are Differentially Modulated by Experience, Time, and Adult Neurogenesis. Neuron 83, 189-201 (2014); Srinivas, S., Watanabe, T., Lin, C.-S., William, C. M., Tanabe, Y., Jessell, T. M. & Costantini, F. Cre reporter strains produced by targeted insertion of EYFP and ECFP into the ROSA26 locus. BMC Dev. Biol. 1, 4 (2001)) transgenic mice. Both male and female mice were used. All mice were group-housed in a 12:12-h (07:00-19:00) light-dark colony room at 22deg°C. Food and water were provided *ad libitum,* and all testing was performed during the light phase. Mice used for experiments were approximately 8-12 weeks of age. All animal work was performed in compliance with laboratory project licenses, with ethical approval from the Trinity College Dublin ethics committee, the Animal Research Ethics Committee from the Health Products Regulatory Authority (HPRA), the Brigham and Women's Hospital Institutional Animal Care and Use Committee guidelines.

### Metabolic Cages

To capture metabolic and behavioral information, mice were singly housed in metabolic cages (Promethion(TM), Sable Systems) for a duration of 8 hours per day. Each cage included a ceiling-mounted food hopper, water bottle and body-mass monitor. Mice had *ad libitum* access to food and water throughout the entire eight hours. Oxygen consumption, carbon dioxide emission, energy expenditure, body weight, food and water intake, and locomotor activity were monitored throughout the session. Briefly, respiratory gasses were measured with an integrated fuel cell oxygen analyzer, a spectrophotometric CO₂ analyzer, and a water vapor pressure analyzer (GA3m1, Sable Systems; Kothari, V., Luo, Y., Tornabene, T., O'Neill, A. M., Greene, M. W., Geetha, T. & Babu, J. R. High fat diet induces brain insulin resistance and cognitive impairment in mice. Biochim. Biophys. Acta Mol. Basis Dis. 1863, 499-508 (2017)). Before each run, gas sensors were calibrated with 100% nitrogen as zero reference and with a span gas containing a known concentration of 0.933% carbon dioxide. Air flow was measured and controlled with the multichannel mass flow generator (FR8-1, Sable Systems). Flow rate was set at 2000 mL/min. Oxygen consumption and carbon dioxide production were measured for each mouse at 3-4 min intervals. Energy expenditure was calculated using the following equation: Kcal/h = 60 * (0.0003941 * VO₂ + 0.001106 * VCO₂; New methods for calculating metabolic rate with special reference to protein metabolism - Weir - 1949 - The Journal of Physiology - Wiley Online Library at physoc.onlinelibrary.wiley.com/doi/10.1113/jphysiol.1949.sp004363).

### Cold Training Schedule

Male mice were taken from their home cage each morning and individually housed in the Promethion(TM) metabolic and behavioral phenotyping cages for a maximum of eight hours. On day one and two, mice were placed into the Promethion(TM) metabolic and behavioral phenotyping cages at 21deg°C to allow them to habituate to the machine. On day three, mice were put into the Promethion(TM) metabolic and behavioral phenotyping cages at 21deg°C while metabolic and behavioral data were simultaneously recorded in order to establish baseline measurements (Context A). On day four, the cages were changed before the mice were placed inside. Contextual cues were added to the chambers, including wall and floor patterns, brighter lights, different bedding and 3 drops of acetic acid odor (Context B). Once the mice were placed into the cages, the temperature decreased in increments of ~10deg°C per hour. As such, the mice spent a total of 6 hours in the altered context at a temperature of 4deg°C. This was repeated for a total of three days, with metabolic and behavioral data recorded throughout. Mice were then returned and kept in their home cages for a two-day break period. Next, on day nine, mice were put back into the Promethion(TM) metabolic and behavioral phenotyping cages without any of the additional cues at 21deg°C to allow for a second baseline measurement (Context A). Finally, on day ten all the additional context cues were added back in before the mice were put into the cages (Context B). Temperature was kept at 21deg°C and metabolic and behavioral data was simultaneously recorded to establish the test day measurements.

### Predator Odor Exposure in Metabolic Cages

Mice were placed into the metabolic cages 24 hours before odor application. 20micro(u)L of either H2O or 10%w/v trimethylthiazole were dropped onto a small 1cm square piece of filter paper in the cage. Mice were exposed to the odorant for 1 hour before being put back into their home cage. Metabolic data was recorded at all times.

### Conditioned Cold Aversion

The conditioned place preference (CPP) apparatus consisted of two separate chambers. Each chamber had different wall and floor cues (black and white pattern). Mice could freely enter both chambers through an opening in the wall in the middle of the apparatus. On day one, mice were taken from their home cage and placed inside the apparatus where they could freely explore both sides of the apparatus for a total of 20 minutes. The entire apparatus was kept at 21deg°C. On day two, the opening in the middle wall of the apparatus was closed. During the morning sessions, mice were exposed to one side of the apparatus at 21deg°C for a duration of two hours. Mice were then returned to their home cage for a two-hour break. In the afternoon sessions, mice were then exposed to the other side of the apparatus at 4deg°C for a duration of two hours. This was subsequently repeated for a total of three days. On day five, the opening in the middle wall of the apparatus was re-opened before mice were placed back into the apparatus. Mice could freely explore both chambers again for a total of 20 minutes, while the entire apparatus was kept at 21deg°C. All behavior was videotaped and subsequently hand scored. Time spent in each chamber in seconds was measured on day one (pre-test) and day five (test). The time spent in each chamber was normalized using the following formula (test phase duration spent in the cold-paired chamber / time spent there in the pre-test phase), to account for any pre-existing preferences (Golden, S. A., Heshmati, M., Flanigan, M., Christoffel, D. J., Guise, K., Pfau, M. L., Aleyasin, H., Menard, C., Zhang, H., Hodes, G. E., Bregman, D., Khibnik, L., Tai, J., Rebusi, N., Krawitz, B., Chaudhury, D., Walsh, J. J., Han, M.-H., Shapiro, M. L. & Russo, S. J. Basal forebrain projections to the lateral habenula modulate aggression reward. Nature 534, 688-692 (2016)). Both time of day and side of apparatus were counterbalanced across all mice.

### Contextual Fear Conditioning

Prior to training, mice were handled for 3 consecutive days in order to habituate them to the experimenter. One day prior to the training day, mice were habituated to the arena and allowed to freely explore the context for 10 minutes. The next day, mice underwent a contextual fear conditioning protocol, where they were allowed to explore the arena for the first 3 minutes. After these 3 minutes, half of the mice received 3 x foot shocks (0.2 s, 0.75mA) that were spaced by intervals of 1 min, whereas the other half of the mice received no foot shocks. Mice were subsequently returned to their home cage for 45 minutes.

### Predator Odor Exposure for BAT harvest

Mice were placed into a vacuum powered 4-chamber olfactory arena, which was replicated from previously published designs (Root, C. M., Denny, C. A., Hen, R. & Axel, R. The participation of cortical amygdala in innate, odour-driven behaviour. Nature 515, 269-273 (2014)). Air entered each corner of the arena at a flow rate of 150ml/minute and was odorized by passing through a bottle containing 20ml of H2O or 350mM trimethylthiazole. Mice were exposed to the odor for 5 minutes and were then sacrificed for tissue collection.

### Tissue Harvest

**Cold memory:** Brains and brown adipose tissue were harvested from mice that went through the above-described Cold Memory timeline. All tissue was harvested on Baseline day 1, Cold day 1, Baseline day 2 and Test day 1. To ensure that the differences seen were due to the differences in conditions and not time of day, all tissues were harvested exactly 5 hours after the mice were put inside the Promethion(TM) metabolic and behavioral phenotyping cages.

**Contextual Fear Conditioning:** For the contextual fear conditioning experiments, brains and brown adipose tissue were harvested 45 minutes after the training session ended.

**Predator Odor Exposure:** For the predator odor exposure experiments, brown adipose tissue was harvested exactly one hour after the animals were exposed to the odors.

### Tissue Preparation

**Brains:** Mice were transcardially perfused with 4% paraformaldehyde (PFA), and brains were fixed in 4% PFA overnight. The next day, brains were transferred into phosphate buffered saline (PBS) and kept at 4deg°C. They were subsequently sliced in 100micro(µ)m coronal sections with a vibratome (Leica VT1200 S) and collected in PBS.

**Brown adipose tissue:** Mice were euthanized by carbon dioxide inhalation and adipose tissue was removed by making a careful incision between the shoulder blades. Interscapular brown adipose tissue was subsequently collected, snap frozen in liquid nitrogen, and stored at -80deg°C.

### Immunohistochemistry

In order to get clear immunolabeling, a previously described iDISCO-based immunohistochemistry protocol was utilized (Renier, N., Wu, Z., Simon, D. J., Yang, J., Ariel, P. & Tessier-Lavigne, M. iDISCO: A Simple, Rapid Method to Immunolabel Large Tissue Samples for Volume Imaging. Cell 159, 896-910 (2014); Pavlova, I. P., Shipley, S. C., Lanio, M., Hen, R. & Denny, C. A. Optimization of immunolabeling and clearing techniques for indelibly labeled memory traces. Hippocampus 28, 523-535 (2018). Sections were rinsed 3 times in 1X phosphate buffered saline (PBS) before subsequently being dehydrated in 50% MeOH/ PBS at room temperature for 2.5 hours. Sections were then rinsed 3 times in 0.2% PBS with TritonX-100 (PBST) and blocked in 0.2% PBST with 10% dimethyl sulfoxide (DMSO) and 6% normal goat serum (NGS) for two hours at room temperature. After blocking, they were washed 3 times in PBS with 0.2% Tween-20 and 10micro(µ)g/ml heparin (PTwH). Sections were then incubated with primary antibody chicken polyclonal anti-GFP (1:1000, Invitrogen, Waltham, MA) and rabbit polyclonal IgG anti-c-Fos (1:1000, Invitrogen, Waltham, MA) at 4deg°C on a shaker for 3 days in PTwH with 5% DMSO and 3% NGS. On day 4, sections were washed 3 times in PTwH and incubated with secondary antibodies Alexa 568 Anti-Rabbit IgG (1:500, Invitrogen, Waltham, MA) and Alexa 488 Anti-Chicken IgG (1:500, Invitrogen, Waltham, MA) overnight at 4deg°C. The next day, sections were washed three times in PTwH, followed by 3 washes in PBS at room temperature. Finally, sections were stained with DAPI in PBS for 10 minutes followed by a final wash in PBS. Sections were then mounted on superfrost slides using Vectashield-DAPI, cover slipped and sealed.

### Confocal Imaging

All images were taken on a confocal scanning microscope (Leica TCS SP8, Leica Microsystems Inc.). Fluorescence from DAPI was detected at 417-488 nm, Alexa Fluor 568 was detected at 595-650 nm, and Alexa Fluor 488 was detected at 500-550 nm. Sections were imaged with a dry 20x objective (NA 0.70, working distance 0.5 mm), with a pixel size of 1.14 x 1.14 micro(µ)m², a z-step of 3 micro(µ)m, and a z-stack of approximately 25 micro(µ)m. Fields of view were stitched together to form tiled images by using an automated stage and the algorithm of the LAS X software.

### Automated Cell Counting

To perform automatic brain wide analysis of c-Fos⁺ cells we used the Neurolnfo software (MBF Bioscience). Briefly, immunohistochemistry-labelled whole brain sections were aligned to the Allen Mouse Brain Atlas using the registration tool within the program (Tappan, S. J., Eastwood, B. S., O'Connor, N., Wang, Q., Ng, L., Feng, D., Hooks, B. M., Gerfen, C. R., Hof, P. R., Schmitz, C. & Glaser, J. R. Automatic navigation system for the mouse brain. J. Comp. Neurol. 527, 2200-2211 (2019)). Here, brain sections were matched to the most closely corresponding atlas plate. All sections were manually adjusted where necessary, to ensure accurate fit. After the correct atlas plate was identified, all anatomical regions of interest were delineated and measured for size in micro(µ)m². Next, c-Fos⁺ cells were identified using the cell detection workflow. Throughout all sections, only cells between 7 micro(µ)m and 19 micro(µ)m were counted for size consistency. These parameters where then combined with a deep learning algorithm to successfully identify c-Fos⁺ cells. Counts were then checked for accuracy and adjusted were necessary. Finally, detected cells were mapped to the Allen Mouse Brain Atlas and tallied in the corresponding anatomical brain structure. Normalized counts (cells per micro(µ)m) were subsequently plotted into Prism 10.0 for further analysis.

### Network Analysis

Cross-correlations between all pairs of regions were calculated in R as previously reported (Santos, S. L., Stackmann, M., Zamora, A. M., Mastrodonato, A., Landri, A. V. D., Vaughan, N., Chen, B. K., Lanio, M. & Denny, C. A. Propranolol Decreases Fear Expression by Modulating Fear Memory Traces. Biol. Psychiatry 89, 1150-1161 (2021)). Pearson's correlations were computed in all cases after which networks were created using the igraph package. To be able to interpret the networks, correlations with an absolute value below 0.5 were not included. Both positive and negative correlations were included in the networks.

### Metabolic Rate and Cell Activity Analysis

In order to investigate whether c-Fos and engram activity correlated with metabolic rate, c-Fos⁺ and co-labeled/eYFP⁺ (%) was correlated with the exact metabolic rate (VO₂, VCO₂, and EE) that was measured 45 minutes prior to the harvesting of the brains.

### RNA Extraction and qPCR Analysis

Harvested brown adipose tissue was defrosted at room temperature and transferred to a 2ml tube and stainless-steel beads were added to each individual tube. Next, tissues were homogenized in 1ml trizol reagent in a tissue lyser for 2.5 minutes at 25 pulses per second. 200 micro(µ)l chloroform was subsequently added to each tube, after which they were inverted and left at room temperature for 3 minutes. All tubes were then centrifuged at 12,000g for 15 minutes, the RNA was transferred into a new tube and 500micro(µ)l isopropanol was added to precipitate the RNA. All tubes were inverted 10 times and left at room temperature for 10 minutes, before being centrifuged again at 12,000g for 10 minutes. Supernatants were then discarded, and all remaining RNA pellets were washed in 1ml of 25% RNAase free dH2O and 75% ethanol. After being centrifuged at 12,000g for 5 minutes, all supernatants were discarded by inverting the tube. RNA pellets were left to dry at room temperature for 20-30 minutes until they were transparent, after which they were re-suspended in 50micro(µ)l RNAase free water. Finally, RNA was left on ice for 30 min and subsequently put on a heat block at 55deg°C for 15 minutes. A Nanodrop 2000 UV spectrophotometer (Thermo Fisher Scientific) was used to assess RNA quality and concentration. 20micro(µ)l of cDNA was subsequently synthesized from 2micro(µ)g of isolated RNA using a cDNA reverse transcription kit (Biosciences Ltd) in a MiniAmp Thermal Cycler (BD Biosciences). qPCRs were then performed to quantify the relative mRNA expression of genes of interest. Relative mRNA levels were calculated using the ΔΔ cycle threshold (ΔΔCt) method and normalized to corresponding endogenous controls (Ppib). All calculated values were plotted into Prism 10 for further analysis.

### Stereotactic Surgeries

**Optic Fiber Implants:** Mice that were used in optogenetic experiments were implanted with a custom implant containing two optic fibers (200 mm core diameter; Doric Lenses). The optical fiber implant was lowered above the injection site at -1.75 mm dorsoventral (DV). To secure the implant, an even layer of Metabond (C&B Metabond) was applied and left to dry for 15 minutes. A protective cap was then made from a black polypropylene microcentrifuge tube and secured with dental cement. Animals were then put in a recovery chamber at 29deg°C until they were fully recovered from the anesthesia before being returned to their home cage. Animals were allowed to recover from surgery ~2-3 weeks before behavioral testing.

**Viral Injections:** c-fos-tTa transgenic mice were put on a diet containing doxycycline (DOX) 48 hours prior to surgeries. All surgeries were performed when the animals were ~7-8 weeks old. Mice were anesthetized with 500 mg.kg-1 avertin and head fixed on a stereotaxic frame. Bilateral craniotomies were performed using a 0.5mm diameter drill at -2.00 mm anteroposterior (AP), and ±1.35 mm mediolateral (ML). Next, 300 micro(u)l AAV9-TRE-ChR2-EYFP was injected in each side using a microsyringe pump (UMP3; WPI) and a Hamilton syringe (701LT; Hamilton) at -2.00 mm anteroposterior (AP), ±1.35 mm mediolateral (ML), and -2.00 mm dorsoventral (DV). Injection speed was performed at 60 nL/min, and the needle was subsequently left on site for an additional 10 minutes after virus delivery to achieve maximum virus spread.

For chemogenetic experiments, TRAP2 mice were injected with 300 micro(µ)L pAAV-hSyn- DIO-HA-hM4D(Gi)-IRES-mCitrine in each side at -2.00 mm anteroposterior (AP), ±1.35 mm mediolateral (ML), and -2.00 mm dorsoventral (DV). Injection speed was performed at 60 nL/min.

### Temperature Probe Surgery

Prior to implantation all temperature probes were sterilized. Mice were anesthetized and positioned on their back as previously described (Santos, S. L., Stackmann, M., Zamora, A. M., Mastrodonato, A., Landri, A. V. D., Vaughan, N., Chen, B. K., Lanio, M. & Denny, C. A. Propranolol Decreases Fear Expression by Modulating Fear Memory Traces. Biol. Psychiatry 89, 1150-1161 (2021)). Next, all the skin around the stomach area was sanitized with 70% ethanol and sterilized saline. A small incision was made using a scalpel in the middle of the abdomen, followed by a 1cm incision in the peritoneum. A temperature probe was subsequently put in the abdominal cavity. Organs were placed over the probe before sewing the peritoneum and skin with sterile stitches.

### Labeling Strategies

**TRAP2 Strategy:** The TRAP2 system allows for the permanent labeling of neurons that were activated by a given experience (Guenthner, C. J., Miyamichi, K., Yang, H. H., Heller, H. C. & Luo, L. Permanent Genetic Access to Transiently Active Neurons via TRAP: Targeted Recombination in Active Populations. Neuron 78, 773-784 (2013)). TRAP2 relies on an immediate early gene locus to drive the expression of iCre recombinase combined with a transgenic Cre-dependent effector. The iCre recombinase can be controlled by tamoxifen. As such, when a neuron is active and tamoxifen is present, the iCre recombinase can enter the nucleus and result in expression of the effector (DeNardo, L. A., Liu, C. D., Allen, W. E., Adams, E. L., Friedmann, D., Fu, L., Guenthner, C. J., Tessier-Lavigne, M. & Luo, L. Temporal Evolution of Cortical Ensembles Promoting Remote Memory Retrieval. Nat. Neurosci. 22, 460 (2019)). The TRAP2 mice were combined with two different reporter lines. For optogenetic experiments, TRAP2 mice were bread with the Ai32 mice (Madisen, L., Mao, T., Koch, H., Zhuo, J., Berenyi, A., Fujisawa, S., Hsu, Y.-W. A., Garcia, A. J., Gu, X., Zanella, S., Kidney, J., Gu, H., Mao, Y., Hooks, B. M., Boyden, E. S., Buzsáki, G., Ramirez, J. M., Jones, A. R., Svoboda, K., Han, X., Turner, E. E. & Zeng, H. A toolbox of Cre-dependent optogenetic transgenic mice for light-induced activation and silencing. Nat. Neurosci. 15, 793-802 (2012)). For engram cell counting experiments, TRAP2 mice were combined with the R26R mice (Srinivas, S., Watanabe, T., Lin, C.-S., William, C. M., Tanabe, Y., Jessell, T. M. & Costantini, F. Cre reporter strains produced by targeted insertion of EYFP and ECFP into the ROSA26 locus. BMC Dev. Biol. 1, 4 (2001)).

To label the cells that were activated during a cold experience, mice were injected with tamoxifen five hours after being put in the Promethion(TM) metabolic and behavioral phenotyping cages on Cold day 1. As such, all cells that were active during the 6-hour cold exposure were labelled. Immediately after labeling all mice were returned to their home cages.

**c-fos-tTa Strategy:** Here, we used an adeno-associated virus (AAV) that expresses Channelrhodopsin-2 (ChR2)-EYFP under the control of a tetracycline-responsive element (TRE)-containing promoter (AAV9-TRE-ChR2-EYFP). The TRE promoter is only active in cells that contain the tetracycline transactivator (tTa) (Hsueh, B., Chen, R., Jo, Y., Tang, D., Raffiee, M., Kim, Y. S., Inoue, M., Randles, S., Ramakrishnan, C., Patel, S., Kim, D. K., Liu, T. X., Kim, S. H., Tan, L., Mortazavi, L., Cordero, A., Shi, J., Zhao, M., Ho, T. T., Crow, A., Yoo, A.-C. W., Raja, C., Evans, K., Bernstein, D., Zeineh, M., Goubran, M. & Deisseroth, K. Cardiogenic control of affective behavioural state. Nature 615, 292-299 (2023)). Therefore, we injected the AAV9-TRE-ChR2-EYFP virus into the dentate gyrus (DG) of c-fos-tTa transgenic mice. These mice express tTa under the control of a *c-fos* promoter (Reijmers, L. G., Perkins, B. L., Matsuo, N. & Mayford, M. Localization of a Stable Neural Correlate of Associative Memory. Science 317, 1230-1233 (2007)). Since c-fos is activity-dependent, the c-fos-tTa transgene will only express tTa in cells that are active during a given experience. By combining the transgenic mice with the virus, it was possible to express tTa and ChR2-EYFP in cells that were active during a cold experience. To have temporal control over this system, mice were put on a diet that contained doxycycline (DOX). DOX is an antibiotic that binds to tTa and as such prevents the expression of the transgene ChR2-EYFP.

To label cells activated by a cold experience, DOX food was substituted by normal food 36h before being exposed to a cold experience. Cold memory engrams were identified as the cells that expressed ChR2-EYFP as a result of being active during the cold. To close the labeling window, mice were immediately put back on DOX food after the initial cold experience.

### 4-hydroxytamoxifen (4-OHT)

To label cold-sensitive engrams in TRAP x Ai32 and TRAP2 x R26R transgenic animals, we intraperitoneally injected mice with 4-OHT (50 mg/kg, Santa Cruz). All 4-OHT was freshly prepared on the labeling day in a mix of sunflower seed oil and castor oil (4:1, Sigma-Aldrich) at 10 mg/ml.

### Engram Tagging

To identify the cells that were active during a cold experience, we used TRAP2 x R26R mice. These mice were chosen as they showed more reliable labeling in the hypothalamus compared to other mice. Briefly, all mice were injected with tamoxifen five hours after being put in the Promethion(TM) metabolic and behavioral phenotyping cages on Cold day 1 to label all cells that were active during the 6-hour cold exposure. Next, mice were exposed to the rest of the cold memory timeline as described above (two additional training days, a two-day break and baseline 2). On test day, half of the mice were put into context B at 21deg°C and half of the mice were kept in their home cage. After exactly 3 hours and 45 minutes all mice were sacrificed to allow for visualization of the immediate early gene c-Fos.

### Engram Counting

All cells were counted using Fiji (Schindelin, J., Arganda-Carreras, I., Frise, E., Kaynig, V., Longair, M., Pietzsch, T., Preibisch, S., Rueden, C., Saalfeld, S., Schmid, B., Tinevez, J.-Y., White, D. J., Hartenstein, V., Eliceiri, K., Tomancak, P. & Cardona, A. Fiji: an open-source platform for biological-image analysis. Nat. Methods 9, 676-682 (2012)). An investigator blind to treatment counted eYFP⁺, c-Fos⁺ and co-labeled cells bilaterally in the Dentate Gyrus (DG), Lateral Hypothalamic Area (LHA), Medial Preoptic Area (MPO) and the Lateral Preoptic Area (LPO). All cell counts were normalized to their respective areas and plotted as cells per micro(µ)m².

### Optogenetic Reactivation of Cold Memories in Metabolic Cages

Shining 472 nm blue light on a ChR2 expressing cell causes ChR2 channels to open, resulting in the depolarization of the neuron. This subsequently enables memory recall to be evoked in mice with ChR2 labelled cells. Optogenetic activation was performed through a 450 nm laser diode fibre light source (Doric LDFLS 450/080). Mice with fiber optic implants were attached to a patch cord (MFP_200/240/900-0.22_0.3m_FC-ZF1.25(F)) and allowed to habituate for 8 hours before being put through the above-described Cold Memory timeline. Cold sensitive engrams were labelled on Cold day 1 and optogenetic reactivation was performed a week later in context A. On the reactivation day, mice were exposed to a 15-minute optogenetic stimulation (8mA) session that was divided into 3-minute on- and 1-minute off periods (i.e. 3 minutes on, followed by 1 minute off). During the light-on epochs, mice received blue light stimulation (20 Hz) with a pulse width of 15ms for the entire 3-minute duration. During the light-off period the mice received no light stimulation. To enable animals to return to baseline, each 15-minute stimulation period was followed by 1 hour of no light stimulation. Mice received a total of 3 rounds of 15-minute stimulation periods. Metabolic rates were measured at all times.

### Chemogenetic Inhibition of Cold Memories in Metabolic Cages

To inhibit cold engrams, we used the same labeling timeline as described above. Briefly, mice went through two habituation days and a baseline measurement day in Context A, at 21deg°C. On day four, contextual cues were added to the chambers, and the mice spent a total of 6 hours in the altered context at a temperature of 4deg°C. To label the neurons that were active during these 6 hours of 4deg°C cold exposure, mice were injected with 4-OHT five hours after being put in the Promethion(TM) metabolic and behavioral phenotyping cages. Immediately after labeling, all mice were returned to their home cages. Mice subsequently went through two additional training days and were subsequently kept in their home cages for a two-day break period. On day nine, mice were put back into the Promethion(TM) metabolic and behavioral phenotyping cages without any of the additional cues at 21deg°C to allow for a second baseline measurement (Context A). Finally, on day ten half of the mice were injected with clozapine-N-oxide (CNO; 2 mg/kg, Tocris) and half of the mice were injected with saline. Mice were kept in their home cage for 30 minutes post injection and were then put into the Promethion(TM) metabolic and behavioral phenotyping cages (Context B, 21deg°C).

### Statistics

All data were analyzed using Prism 10. All data were graphed as means ± Standard errors of the mean (SEM). An alpha level of 0.05 was used as a criterion for statistical significance, and probability levels were quoted for non-significance.

### Figure design and visualization

Figures and graphical illustrations were created with Biorender (Biorender.com).

### Example 1

### Cold memories increase metabolism

To investigate whether mice can store and retrieve temperature information, a thermoregulatory Pavlovian conditioning paradigm was designed where mice were trained in Context A at 21deg°C and in Context B at 4deg°C (Figure 1a-b). Cold exposure stimulates thermogenesis by increasing lipid and glucose metabolism in BAT, which in turn upregulates whole-body metabolic rate. Mice exposed to a 4deg°C environment had a lower core body temperature compared to when they are exposed to a 21deg°C environment (Figure 6a-f) and showed increased oxygen consumption (VO₂; Figure 7a), energy expenditure (EE; Figure 7b) and carbon dioxide production (VCO₂; Figure 7c).

Mice also moved significantly more in the 4deg°C environment than in the 21deg°C environment (Figure 7d). No differences in food consumption were observed (Figure 7e). It was additionally found that the observed increase in metabolic rate and energy expenditure persisted across all three cold training days (Figure 7a-c). Together, these data show that mice increase their metabolic rate, and alter their behavior, in response to a cold environment.

To establish whether mice can associate a specific context with a given temperature, mice were trained at 4deg°C in Context B and subsequently tested in that same context at 21deg°C. An increase in metabolic rate was observed when mice were returned to the cold-paired context, irrespective of the actual temperature of that context (Figure 1c-g, Figures 7a-b, 8a-e). This effect was specifically induced by the contextual cues associated with cold training, and not just exposure to a neutral context at baseline day 2 (BL2; Figures 7a-e, 9f-m). Furthermore, when mice were returned to the cold-paired context on test day 1 (T1), they increased their core body temperature compared to baseline day 1 (BL1; Figure 6b-j). In addition, mice showed significantly increased movement when returned to the cold-paired context (Figure 8c), but no difference in food consumption compared to baseline day (Figure 8d), suggesting that mice can adjust their behavior in the cold-paired context on test day, similar to what is observed in the 4deg°C context. Although differences in movement were found across various days, the observed increase in metabolic rate on test day was not solely driven by movement (Figure 9n-q). Interestingly, a temporal dynamic was also found on test day, as the metabolic rates resemble cold day 1 during the first few hours and come closer to baseline levels after 6 hours (Figure 1c-g). Both males and females increased their metabolic rate when they were returned to the cold-paired context (Figures 9f-m, 10a-c).

To confirm that the upregulation of metabolic rate on test day was due to the pairing of 4deg°C with Context B, and not due to novelty of the context, we trained mice in the same Pavlovian timeline without deviating the temperature from 21deg°C (Figure 1h-i). It was found that, although the novelty of Context B caused an increase in metabolic rate in the initial 2 hours of the no-cold training and test days, there were no differences in metabolic rate between baseline, no-cold training and test after two hours (Figure 1j), suggesting that novelty does not affect metabolic rate beyond two hours. Mice that underwent 4deg°C cold training in Context B were directly compared with mice that underwent the 21deg°C no-cold training in Context B. These results demonstrate that cold training at 4deg°C in Context B significantly increased metabolic rate on test day 1 compared to 21deg°C no-cold training (Figure 1k). These effects are not due to initial differences between the two groups, as they did not significantly differ in metabolic rate on baseline day (Figure 11).

These data show that the increase in metabolic rate is not caused by the novelty of Context B, but rather by the association of 4deg°C with Context B. To rule out that the increase in metabolic rate was due to prolonged effects of long-term cold exposure, a separate cohort of mice were allowed to recover in their home cage for four consecutive days instead of two after cold training (Figure 11a-g). It was found that the increase in metabolic rate persisted even after this prolonged recovery break, indicating that the observed increase in whole-body metabolism was not due to prolonged cold effects, but rather to the recall of the cold-paired contextual memory. To rule out that the increase in metabolic rate was due to a generalized stress response, mice were exposed to the predator odor trimethylthiazoline (TMT) while simultaneously recording metabolic rates. No increase in metabolic rate in mice that were exposed to TMT was observed, compared to mice that were exposed to a control odor (Figure 11h,i). These data suggest that the increase in metabolic rate on T1 was not due to a general stress response, but to the recall of a cold memory specifically.

Finally, to demonstrate the locomotory behavioral expression of a cold memory, it was asked whether mice develop a conditioned cold aversion due to training at 4deg°C in a conditioned place preference (CPP) apparatus (Figure 11j). It was shown that mice have no preference for either side of the apparatus during the pre-test day at 21deg°C (Figure 11k). However, mice trained at 4deg°C displayed a conditioned place aversion to the cold-paired side of the CPP apparatus but not to the 21deg°C paired side (Figure 11l). These data demonstrate that mice can form a contextual memory for a cold environment, which upon retrieval leads to an increase in whole-body metabolism and avoidance behavior.

### Example 2

### Cold memories upregulate thermogenesis

Brown adipose tissue (BAT) is a specialized tissue that is important for non-shivering thermogenesis in both humans and rodents. The lipolysis metabolic pathway has been demonstrated to fuel the thermogenic response in brown adipocytes via uncoupling protein 1 (UCP-1; Figure 1m). To further establish the relationship between cold memory and whole-body metabolic responses, BAT was harvested from mice at different time points on the experimental schedule (Figure 1m). It was shown that after cold exposure, multiple thermogenic genes are upregulated, including *Ucp1* (Figure 12b-g). Strikingly, it was found that several thermogenic genes are also upregulated in mice during cold recall on T1, compared to control mice on BL2 (Figure 1n-s). Indeed, an upregulation of *Ucp1* (Figure 1n) was observed, in addition to an increase in mitochondrial genes involved in fatty acid uptake, such as *Cpt1alpha(α)* and *Cact* (Figure 1o,p) and genes associated with fatty acid release from lipid droplets, such as *Atgl* and *Hsl* (Figure 1q,r). These results show that recall of a cold memory is sufficient to upregulate thermogenic genes in BAT.

It was tested whether the observed upregulation of thermogenesis genes was specifically due to cold experiences or due to a generalized stress response. BAT was harvested from animals that went through contextual fear conditioning training and compared with animals that received no foot shocks (Figure 12h). No differences were observed in any of the examined thermogenesis genes between shocked mice and no shock controls (Figure 12i-n). Next, BAT was harvested from mice that were exposed to a predator odor (TMT) or to a neutral control odor (H2O, Figure 12o). No increase in thermogenic gene expression was found in mice exposed to TMT compared to mice that were exposed to H2O (Figure 12p-u). However, significantly decreased expression of *Hsl* was found in TMT exposed mice compared to H2O controls (Figure 12t). Together, these experiments showed that thermogenesis genes in BAT are not upregulated due to a stressful experience in general, but that this increase is due to a cold experience specifically. It can therefore be concluded that cold memories can control whole-body thermoregulatory responses.

### Example 3

### Cold memories enhance HPC and HY activity

To identify the neuronal activity patterns underlying cold memory encoding and retrieval, c-Fos expression was examined throughout multiple brain regions. c-Fos activity was compared during baseline (BL1), cold training day 1 (CL1) and test day (T1), to determine which regions were active during specific conditions (Figure 2a). To enable automated c-Fos detection in various hippocampal and hypothalamic regions, all brains were run through an automated brain-wide counting pipeline (Figure 2b,c). These results show no differences in the number of c-Fos⁺ cells in the dentate gyrus (DG) between any of the three examined conditions (Figure 2d). However, cold-activated c-Fos activity was found in the cornu Ammonis 3 (CA3) and cornu Ammonis 1 (CA1) subregions of the hippocampus at CL1 and T1, compared to BL1, suggesting that CA3 and CA1 are important areas that are involved in cold responses (Figure 2e,f). Although no innate differences in c-Fos⁺ activity were found in the DG between the three conditions, it is hypothesized that there may be differences in how this region interacts with whole-body metabolism after learning. As such, c-Fos activity in the DG was correlated with oxygen consumption (VO₂) at BL1, CL1, or T1 (Figure 2g). These results show that, at BL1, there was no correlation between c-Fos activity in the DG and metabolic rate. At CL1, there was a negative correlation between VO₂ and DG activity. However, at T1, after the cold memory has been consolidated, there is a significant positive correlation between VO₂ and DG activity (Figure 2g). A similar correlation pattern was observed in CA1 but not in CA3 (Figure 13a,b). It can be concluded that various subregions of the hippocampus are involved in the encoding or retrieval of contextual cold memories, and that the DG and CA1 play a role on T1 in regulating whole-body metabolism.

c-Fos activity was compared in lateral hypothalamic area (LHA), medial preoptic area (MPO), and lateral preoptic area (LPO) key brain regions at BL1, CL1 and T1. It was shown that c-Fos activity in the LHA is upregulated when mice are exposed to the cold during CL1 (Figure 2h). Interestingly, similar levels of activity were found in this area when mice are recalling the cold on T1. Although increased activity in the MPO and LPO is seen during cold exposure (Figure 2i,j), no increases in c-Fos activity were found in these areas on T1. Moreover, no increases in c-Fos activity were found at CL1 or T1 in any of the other investigated hypothalamic areas (Figure 13h-k). Next, it was tested whether the LHA, MPO and LPO correlated with metabolic rate. It was found that only the mice that were exposed to the cold on CL1 show a positive correlative trend between c-Fos activity in the LHA and oxygen consumption (Figure 2k). This trend was not found in any other condition or hypothalamic region (Figure 13c-d). These findings suggest that the LHA is important during encoding and recall of cold, and that it correlates with metabolic rate during a cold experience. Finally, to confirm that the increases in c-Fos activity found during CL1 and T1 were due to cold exposure and recall specifically rather than stress, mice were trained in a fear conditioning paradigm (Figure 14a). It was shown that mice that underwent contextual fear conditioning training do not show similar increases in c-Fos activity in hippocampal or hypothalamic regions, as observed in the cold (Figure 14c-j). Additionally, increased c-Fos was observed in amygdalar regions following fear conditioning, but not during the cold or cold recall (Figure 13m-o; 9l-n). These data suggest that stress was not the cause of the neural activity that was found in the hippocampus or hypothalamus.

To understand how functional connectivity between brain regions is altered during cold exposure and cold memory retrieval, further correlational analyses of c-Fos activity were performed across brain regions (Figure 13r-t). From this, correlational networks were built that corresponded to BL1, CL1, and T1 to further visualize plasticity of functional connectivity (Figure 2l-n). It was found that, compared to the baseline network, there is increased functional connectivity between hypothalamic regions when mice are exposed to the cold (Figure 2l,m). Moreover, an increase in functional connectivity between the hippocampus and the hypothalamus was found when mice are recalling the cold, compared to when they are experiencing the cold, which is not observed in the baseline network (Figure 2l-n). These data suggest that hippocampus to hypothalamus connectivity is a plasticity feature that forms after mice encode a cold contextual memory.

### Example 4

### Cold engrams are in the DG, LHA, and MPO

Engram ensembles have been identified in many different areas of the brain, such as the hippocampus, amygdala and prefrontal cortex for various sensory modalities. To better understand how cold memories are encoded and retrieved, TRAP2 mice were crossed with R26R-STOP-floxed enhanced yellow fluorescent protein (eYFP) transgenic mice to achieve optimal labeling in the DG, LHA, MPO, and LPO (Figure 3a).

To quantify activated engram cells in multiple brain regions, mice were put through a cold engram labeling protocol (Figure 3b). The number of eYFP⁺ cells was first quantified in the DG, LHA, MPO, and LPO (Figure 3c). These results show no differences in the number of eYFP⁺ cells between mice recalling a contextual cold memory at T1 and home cage controls in the DG, LHA, MPO, or LPO, suggesting comparable amounts of labeled cells in both groups (Figure 3d-g). Next, the number of c-Fos⁺ cells was quantified between the groups (Figure 3h-l). There was a significant increase in the number of c-Fos⁺ cells in mice retrieving the cold in the LHA (Figure 3j), whereas no significant differences were found in the DG, MPO, or LPO (Figure 3i, k-l). Finally, engram reactivation was measured by quantifying the cells active during both encoding and expression of a cold memory (i.e., ((c-fos⁺eYFP⁺)/eYFP⁺; Figure 3m). These results show a significant increase in co-labeling in the DG, LHA and MPO (Figure 3o-p), but not in the LPO (Figure 3q) in mice retrieving a cold memory when compared to home cage controls. Together, these data suggest that cold-sensitive memory engrams are located in the DG, LHA, and MPO.

It was next analyzed how increased reactivation of cold-sensitive engrams affected whole-body metabolism. Here, the amount of co-labeled cells in the DG was correlated with VO₂, VCO₂, and EE. Strong positive correlations were found between cold engram cell reactivation in the DG and metabolic rate at T1 and CL1, but not at BL1 (Figure 3r-t). These results show that increased cold engram reactivation during recall covaries with whole-body metabolism during both recall, and crucially, with metabolism during the original training day when the engram was formed. Finally, correlational analyses of engram reactivation activity across brain regions were performed. These analyses revealed a significant positive correlation between the DG and LHA in animals recalling the cold (Figure 3u), whereas this type of coordinated activity was not found in the home cage control animals (Figure 3v). Similarly, we find coordinated activity between the MPO and LPO in test animals but not in controls (Figure 3u-v). These results suggest that when mice recall the cold, a specific engram pathway emerges between the DG and LHA, in addition to a connection between the MPO and LPO, and that coordinated hypothalamic activity is essential for contextual cold memories.

### Example 5

### Cold engram stimulation increases metabolism

To determine whether cold-sensitive engrams are functionally relevant for whole-body metabolism, it was set out to optogenetically reactivate DG cold engrams. To do so, cold-sensitive engrams in the DG were labelled on cold day 1, and optogenetically reactivated in a neutral context (Figure 4a-d, Figure 15a-c). It was shown that optogenetic reactivation of cold-sensitive engrams in the DG is sufficient to artificially increase whole-body metabolism (Figure 4e, Figure 16j,l). Moreover, it was found that metabolic rates increased when cold-sensitive engrams were stimulated, and that they returned to baseline during the unstimulated period (Figure 4f-i). Of note, metabolic rate increased only in the first two consecutive stimulations, but not during a third stimulation, suggesting an attenuation of whole body-responses or a limitation of optogenetic engram stimulation (Figure 4h). Additionally, we demonstrate that optogenetic reactivation of cold-sensitive engrams is possible with multiple transgenic mouse lines, and with different engram labeling strategies including one based on the c-fos-tTA system (Figure 17a-h).

To confirm that the increase in metabolic rate was due to contextual cold memories, a control experiment was designed where mice were run through the same timeline but were never exposed to the cold (Figure 16a,b). As such, no-cold engram cells in the DG were labelled and optogenetically reactivated in a neutral context. These results show that optogenetically stimulating no-cold engram cells does not increase whole-body metabolism (Figure 16c,k,m). There were no significant differences between on and off periods, during any of the three stimulations (Figure 16d-g). Interestingly, a slight decrease in oxygen consumption was found when the laser was on compared to when it was off (Figure 16g). Next, optogenetic stimulation of cold-sensitive engrams was directly compared with "no-cold" contextual engrams (Figure 16h). It was shown that oxygen consumption significantly increased in the cold labeled group compared to the no-cold labeled group during laser stimulation (Figure 16i). The difference in metabolic rate during laser activation was not due to pre-existing differences between the cohorts, as no differences were found between the cold and no-cold labeled groups when the laser was off (Figure 16i). Therefore, it can be concluded that cold-sensitive engrams control whole-body metabolism, as optogenetic activation of these cells increases metabolic rate.

It was investigated whether optogenetic stimulation of cold-sensitive DG engrams would result in increased engram activity in hypothalamic regions. To achieve this, cold engrams in the DG were labelled with ChR2 on cold day 1, whilst labelling engrams throughout the brain with eYFP on a background of the TRAP2/R26R compound transgenic line. Artificially reactivated engram cells in the LHA, MPO and LPO were subsequently quantified (Figure 4j-i). It was shown that there was a significant increase in co-labeling (i.e., ((c-fos⁺eYFP⁺)/eYFP⁺) in the LHA and MPO after optogenetic stimulation of cold-sensitive DG engrams compared to after stimulation of no-cold engrams (Figure 4m-o, Figure 17i-r). This increase in co-labeling was not found in the LPO (Figure 4o), suggesting that only specific regions of the hypothalamus have increased engram connectivity with the DG. Importantly, it was found that the artificially induced engram activity in the LHA significantly correlated with VO₂ (Figure 4p), suggesting that the connectivity found between the DG and specific regions of the hypothalamus is mediating the learned control of whole-body metabolism.

Next, cold-sensitive engrams were optogenetically reactivated to artificially induce a thermogenic response, and BAT was harvested to determine whether artificial reactivation of cold memories increases thermogenic gene expression in adipose tissue (Figure 4q). These results show that artificial reactivation of a cold-sensitive engram leads to an increase in *Ucp1* (Figure 4r) and *Cpt1alpha* (Figure 4s) expression in BAT, compared to the reactivation of no-cold engram cells. No differences were found in other thermogenic genes (Figure 4t-v). Together, these results show that both natural and artificial retrieval of cold memories causes an upregulation of thermogenesis genes in BAT.

### Example 6

### Inhibiting cold engrams blocks metabolic change

To further explore the role of cold-sensitive engram cells, DG cold engrams were chemogenetically inhibited during cold memory recall. To do so, cold-sensitive engrams were labeled in the DG of the hippocampus on CL1 with inhibitory DREADD hM4Di receptors and chemogenetically inhibited these cells by injecting clozapine-N-oxide (CNO) on T1 (Figure 5a-c, Figure 15d-g). An increase in metabolic rate was observed on T1 compared to BL1 when saline was injected into control animals on T1. This increase in metabolic rate was observed throughout the two hours, and for the combined total testing time (Figure 5d-g; Figure 18b,c,g,h). In contrast, it was found that CNO-treated mice no longer showed the learned increase in metabolic rate on T1 (Figure 5h). Indeed, it was found that the conditioned response was no longer present at any point throughout the testing time (Figure 5h-k; Figure 18d,e,i,j). When directly comparing the saline and CNO injected mice, a significantly higher percentage of change in metabolism was found in the saline injected mice compared to the CNO injected mice (Figure 5l-n; Figure 18f,k). Together, these data show that by inhibiting cold-sensitive cells in the DG, contextual cold memories could be inhibited and, as a result, prevent the conditioned whole-body responses that were previously associated with these contextual stimuli.

Environmental thermal challenges trigger the brain to coordinate both autonomic and behavioral responses to maintain optimal body temperature. It is unknown how temperature information is precisely stored and retrieved in the brain, and how it is converted into a physiological response. Here, it was investigated whether memories could control whole-body metabolism by training mice to remember a thermal challenge. Mice were conditioned to associate a context with a specific temperature, by combining thermoregulatory Pavlovian conditioning with engram labeling technology, optogenetics, and chemogenetics. It is shown that, if mice are returned to an environment where they previously experienced a 4deg°C cold challenge, they increase their metabolic rates regardless of the actual environmental temperature. Additionally, increased hypothalamic activity was shown when animals are exposed to the cold, and that a specific network emerges between the hippocampus and the hypothalamus during the recall of a cold memory. Both natural retrieval and artificial reactivation of cold-sensitive memory engrams in the hippocampus mimic the physiological responses that are seen during a cold-challenge. Finally, these ensembles are necessary for cold memory retrieval. These results show that retrieval of a cold memory causes whole-body autonomic and behavioral responses that enable animals to maintain thermal homeostasis.

In this study, the influence of memory engrams on whole-body responses was investigated. It was discovered that mice increase their metabolic rate, core body temperature, and adjust their behavior when recalling a cold environment, indicating that mice can store and recall temperature information. These findings reveal that the recall of a cold environment increases c-Fos activity in the hippocampus and hypothalamus, and that it leads to increased functional connectivity between these two structures. It was found that cold-sensitive engram cells are located in the DG, LHA and MPO and that artificial reactivation of DG engram cells leads to an increase in whole-body metabolism and engram cell activity in the hypothalamus. Moreover, these results showed that inhibition of cold-sensitive cells in the DG prevents the conditioned whole-body response that was previously associated with cold memories. Notably, it was shown that contextual cold memories control whole-body responses in BAT, by increasing thermogenic genes during cold recall. Together, these data show that cold sensitive engrams control whole-body thermoregulatory responses.

This is the first study implicating memories with changes in in whole-body metabolism. The increases found here are not solely due to a general stress response to the cold, as it was found that exposure to other stressful experiences did not increase metabolic rate, or expression of lipolytic and thermogenic genes. Although cold exposure may inevitably cause some stress to mice, it does not explain the memory outcomes found here. No differences were found in food consumption during cold exposure or cold memory recall, suggesting that eight hours of cold exposure is not long enough to cause changes in feeding behavior. Interestingly, it was found that animals increased their movement across training days and when recalling the cold, indicating that previous experience and expectations causes adaptive behavioral responses in mice.

No differences were found in c-Fos activity in the DG after cold exposure. However, significant increases in neuronal activity in CA3 and CA1were found, suggesting that both these areas potentially hold innate cold sensing cells. By comparing the cells that were active during the encoding and recall of a cold memory, putative cold-sensitive memory engrams were found in the DG, LHA and MPO. Previous studies have shown that engram cells are necessary and sufficient to induce the learning-specific conditioned behavioral response, and that neural activity can regulate immune responses. Here, it is described how engram cells control peripheral physiological processes beyond traditionally studied behavioral responses. These results show that contextual cold engrams in the DG control whole-body metabolism through engram connectivity in the LHA and MPO. Indeed, in this study, it is shown for the first time that engram connectivity in the hypothalamus resulting from artificial DG stimulation correlates with whole-body metabolism.

BAT has been found to be a versatile organ that is involved in a variety of metabolic and endocrine processes. Recently it has also been suggested that it might be a stress-responsive organ that is crucial for a "fight or flight" response. Here, the genes in the thermogenic pathway analyzed in this study are not uniquely activated by stress. Indeed, these results showed no differences in thermogenic gene expression in fear conditioned or predator odor exposed mice. As such, it has been shown that these genes are specifically involved in thermoregulatory processes that are activated when an animal is encoding and recalling a cold environment. Together, these data seem to suggest that different types of stressors elicit different responses from BAT, and that its response is highly dependent on the type of contextual input that it receives.

It has been shown that animals can be conditioned to associate a particular context with a cold temperature experience. A Pavlovian conditioned thermoregulatory response can thereafter be elicited by exposure to contextual stimuli associated with cold experiences. It was also found that hypothalamic activity is highly upregulated when animals are exposed to the cold, and that a specific network emerges between the hippocampus and the hypothalamus during the recall of a cold memory. It was shown that cold-sensitive memory engrams form in the hippocampus and specific regions of the hypothalamus, and that both natural retrieval and artificial reactivation of these engrams mediate the physiological responses that are seen during a cold-challenge. Furthermore, these ensembles are necessary for the normal retrieval of a cold memory. These results highlight that cold-sensitive engrams control whole-body autonomic and behavioral responses that enable animals to maintain thermal homeostasis.

Here, it was shown that by stimulating engram cells in the brain, it is possible to upregulate thermogenesis genes in BAT.

## Claims

1. A method for inducing thermogenesis in a subject, the method comprising the steps of:
(a) exposing the subject to a first temperature below 21deg°C;
(b) simultaneously exposing the subject to a stimulus at the first temperature; and
(c) exposing the subject to the stimulus at a second temperature different to the first temperature;
thereby inducing thermogenesis in the subject at the second temperature.

2. A method according to claim 1, wherein the method comprises the steps of:
(a) providing to the subject a first temperature below 21deg°C;
(b) simultaneously providing to the subject a stimulus at the first temperature; and
(c) providing to the subject the stimulus at a second temperature different to the first temperature;
thereby inducing thermogenesis in the subject at the second temperature.

3. A method according to claim 1 or 2, wherein the method is a non-therapeutic method for inducing weight loss in the subject.

4. A method according to claim 1 or 2, wherein the method is a cosmetic method for inducing weight loss in the subject.

5. A method according to any one of claims 1-4, wherein the first temperature is below 5deg°C.

6. A method according to any one of claims 1-5, wherein the exposing the subject to a first temperature step comprises decreasing the temperature at a rate of 10deg°C per hour.

7. A method according to any one of claims 1-6, wherein the exposing the subject to a first temperature step occurs for at least 6 hours.

8. A method according to any one of claims 1-7, wherein the stimulus is selected from a visual stimulus, an auditory stimulus, a somatosensory stimulus, an olfactory stimulus, a gustatory stimulus, and combinations thereof.

9. A method according to any one of claims 1-8, wherein the exposing the subject to a first temperature step occurs for the same period as the simultaneously exposing the subject to a stimulus at the first temperature step.

10. A method according to any one of claims 1-9, wherein the second temperature is above the first temperature.

11. A method according to any one of claims 1-10, wherein the method comprises the steps of:
(a) providing to the subject, or exposing the subject to, a first temperature of 4deg°C;
(b) simultaneously providing to the subject, or exposing the subject to, a stimulus at the first temperature of 4deg°C; and
(c) providing to the subject, or exposing the subject to, the stimulus at a second temperature of 21deg°C;
thereby inducing thermogenesis in the subject at the second temperature of 21deg°C.

12. A method according to any one of claims 1-11, wherein the method comprises the steps of:
(a) providing to the subject, or exposing the subject to, a temperature of or above 21deg°C;
(b) providing to the subject, or exposing the subject to, a first temperature below 21deg°C;
(c) simultaneously providing to the subject, or exposing the subject to, a stimulus at the first temperature; and
(d) providing to the subject, or exposing the subject to, the stimulus at a second temperature different to the first temperature;
thereby inducing thermogenesis in the subject at the second temperature.

13. A method according to any one of claims 1-12, wherein the thermogenesis is non-shivering thermogenesis.

14. A method according to any one of claims 1-13, wherein the thermogenesis is induced in a brown adipose tissue.

15. A method according to any one of claims 1-14, wherein the method increases the average/mean core body temperature of the subject.
